# EUROPEAN PATENT SPECIFICATION

(11) **EP 4 289 934 B1**
(45) Date of publication and mention of the grant of the patent: **31.12.2025**
(21) Application number: 22178172.7
(22) Date of filing: 09.06.2022
(51) Int. Cl.: C12M 3/06, C12M 1/12

(54) **CELL CULTURE MEMBRANE STRUCTURE, METHODS FOR PRODUCING THE SAME, CELL CULTURE PLATE, CELL CULTURE APPARATUS COMPRISING THE CELL CULTURE MEMBRANE STRUCTURE, AND METHODS FOR CELL CULTIVATION BY USING THE CELL CULTURE APPARATUS**
ZELLKULTURMEMBRANSTRUKTUR, VERFAHREN ZU DEREN HERSTELLUNG, ZELLKULTURPLATTE, ZELLKULTURGERÄT MIT DER ZELLKULTURMEMBRANSTRUKTUR UND VERFAHREN ZUR ZELLKULTIVIERUNG UNTER VERWENDUNG DES ZELLKULTURGERÄTES
STRUCTURE DE MEMBRANE DE CULTURE CELLULAIRE, SES PROCÉDÉS DE PRODUCTION, PLAQUE DE CULTURE CELLULAIRE, APPAREIL DE CULTURE CELLULAIRE COMPRENANT LA STRUCTURE DE MEMBRANE DE CULTURE CELLULAIRE ET PROCÉDÉS DE CULTURE CELLULAIRE À L'AIDE DE L'APPAREIL DE CULTURE CELLULAIRE

(43) Date of publication of application: 13.12.2023
(73) Proprietor: Finnadvance Oy, 90220 Oulu (FI)
(72) Inventor: Singh, Prateek, 90220 Oulu (FI); Nguyen, Hoang Tuan, 90220 Oulu (FI)
(74) Representative: Papula Oy

(56) References cited:
- WO-A2-2018/226902
- US-A1- 2005 003 524
- US-A1- 2008 138 900
- US-A1- 2020 095 531
- US-A1- 2021 340 477

## Description

### TECHNICAL FIELD

The present disclosure relates generally to the field of biotechnology. In particular, the present disclosure relates to a cell culture membrane structure, methods for producing this structure, as well as a cell culture plate and a microfluidic device using this structure.

### BACKGROUND

Cell culture or cultivation involves growing cells, organoids, and/or spheroids of desired type(s) outside a living body under controlled *in vitro* conditions to sustain growth and viability of the cells, organoids, and/or spheroids. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies.

Environmental conditions created for cultured cells should resemble as closely as possible the conditions experienced by the same cells *in vivo.* This may be done by performing the cell culture in large vessels, such as dishes, spinner and shaker flasks. However, the cell culture conditions provided by these vessels do not truly represent the *in vivo* environment of the cells being cultured. Moreover, since these vessels have large volumes, they consume significant amounts of reagents, culture media, chemicals etc., thereby making it difficult to control and/or alter the cell culture conditions. On top of that, these vessels are generally unable to provide uniform cell culture conditions to all the cells throughout the volume of the vessel. US 2020/095531 shows a membrane with through pores and non passing cavities as well as coatings selected for instance among fibronectin, collagen genalin, laminin etc.. US 2005/003524 refers to a porous membrane with open porous structure and hollow fiber capillary membranes and coating processes. US 2008/138900 discloses a coated membrane with cavities for allocating cells and through pores. WO 2018 226902 also discloses porous membranes with through-holes coated with different selected molecules.

At least some of the above-listed drawbacks may be eliminated by using thin, porous membranes as cell culture supports. In particular, the porous membrane provides improved control over the cell culture conditions. The porous membranes also enable parallel culturing of a large number of cells under the same or similar cell culture conditions. In general, the porous membranes enable the partitioning of cellular microenvironments and cell types in *vitro,* while still allowing physical and biochemical crosstalk between cells.

However, a limiting factor of using the thin, porous membranes is that they need to be strong enough to withstand forces applied thereto during their production and subsequent manipulations. At the same time, the room for adjusting membrane properties for the cell culture is very limited. In many cases, the porous membranes cannot be adapted for a specific cell culture.

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a cell culture membrane structure that is adapted for specific cells, organoids, and/or spheroids to be cultured thereon.

The objective above is achieved by the features of the independent claims in the appended claims. Further embodiments and examples are apparent from the dependent claims, the detailed description, and the accompanying drawings.

Disclosed is a cell culture membrane structure comprising:
- a membrane made of a membrane material, the membrane comprising:
- a first surface being partially deformed by one or more membrane structure cavities protruding from the first surface, and
- one or more through pores formed therein;
wherein the membrane material is selected based on at least one type of cells to be cultured on the membrane.

The present disclosure also provides a cell culture plate comprising:
a housing encompassing one or more wells, each well being arranged to retain a cell culture media, and
one or more cell culture membrane structures as defined in the present disclosure each being a replaceable insert configured to be immersed into the cell culture media such that each of the inner insert cavities of each of the replaceable inserts is in flow communication with one well of the housing via the through pores.

The present disclosure also provides a cell culture apparatus comprising:
one or more cell culture modules, each cell culture module of the one or more cell culture modules comprising:
   - at least two culture medium reservoirs each having a top part and a bottom part, the top part having an inlet for a culture medium and the bottom part having an outlet for the culture medium;
   - a first chamber configured to be arranged between the at least two culture medium reservoirs such that the first chamber and the at least two culture medium reservoirs are aligned with each other in a first direction, and
   - a cell culture membrane structure as defined in the present disclosure configured to be arranged between the first chamber and a second chamber such that the first chamber and the second chamber are in flow communication with each other via the through pores; or
   - a cell culture membrane structure as defined in the present disclosure configured to be arranged between the at least two culture medium reservoirs such that the cell culture membrane structure and the at least two culture medium reservoirs are aligned with each other in a first direction, the membrane of the cell culture membrane structure being arranged between the inner insert cavity and a second chamber such that the inner insert cavity and the second chamber are in flow communication with each other via the through pores;
wherein the second chamber being either arranged under and aligned with the first chamber, or arranged under and aligned with the cell culture membrane structure as defined in the present disclosure, in a second direction, the second direction having an angle being more than 0 degrees but 90 degrees or less in respect to the first direction, the second chamber having a bottom part having at least two lateral holes; and
   - at least two flow channels, each of the at least two flow channels being configured to pass a fluid or cell culture media therethrough, and each of the at least two flow channels connecting the outlet of the bottom part of one of the at least two culture medium reservoirs to one of the at least two lateral holes of the bottom part of the second chamber.

The present disclosure also provides a cell culture incubator comprising:
the cell culture apparatus as defined in the present disclosure; and
a pipetting station configured to feed the culture medium to each of the at least two culture medium reservoirs in each cell culture module of the one or more cell culture modules.

The present disclosure also provides a cell culture incubator comprising:
the cell culture apparatus as defined in the present disclosure; and
a pipetting station configured to feed the culture medium to each of the at least two culture medium reservoirs in each cell culture module of the one or more cell culture modules.

The present disclosure also provides a method for producing a cell culture membrane structure, comprising:
- providing a membrane made of a membrane material;
- forming one or more through pores in the membrane;
- deforming the membrane comprising one or more through pores formed therein, thereby obtaining one or more membrane structure cavities protruding from a first surface of the membrane;
wherein the membrane material is selected based on at least one type of cells to be cultured on the membrane.

The present disclosure also provides a method for producing a cell culture membrane structure, comprising:
- providing a membrane made of a membrane material,
- applying at least one coating layer on the membrane in a continuous manner, the at least one coating layer being made of a coating material; and
- forming through pores coming through the at least one coating layer and the membrane;
wherein the membrane material and the coating material are selected based on at least one type of cells to be cultured on the at least one coating layer.

The present disclosure also provides a method for cell cultivation by using the cell culture apparatus as defined in the present disclosure, the method comprising:
(a) providing the culture medium to one of the at least two culture medium reservoirs in each cell culture module of the one or more cell culture modules; and
(b) causing the culture medium to flow from said one of the at least two culture medium reservoirs to the rest of the at least two culture medium reservoirs in each cell culture module of the one or more cell culture modules for a predefined time period, the predefined time period being selected based on the culture medium.

The present disclosure also provides a method for cell cultivation by using the cell culture apparatus as defined in the present disclosure, the method comprising:
(a) providing the culture medium to one or more of the at least two culture medium reservoirs in each cell culture module of the one or more cell culture modules;
(b) causing the culture medium to flow to the second chamber from said one or more of the at least two culture medium reservoirs in each cell culture module of the one or more cell culture modules;
(c) placing the cell culture apparatus in an inverted position; and
(d) incubating the cell culture apparatus in the inverted position for a predefined time period, the predefined time period being selected based on the culture medium.

### BRIEF DESCRIPTION OF THE DRAWINGS

The present disclosure is explained below with reference to the accompanying drawings in which:
FIGs. 1A-1B show different schematic views of a cell culture membrane structure in accordance with exemplary embodiments, namely: FIG. 1A shows a close up of a schematic perspective view of a cell culture membrane structure in accordance with an exemplary embodiment, FIG. 1B shows a schematic cross-sectional side view of a cell culture membrane structure in accordance with an exemplary embodiment;
FIG. 2 shows a flowchart of a method for producing the cell culture membrane structure shown in FIG. 1A;
FIG. 3 explains how to apply a dual coating on the structure shown in FIG. 1A by using a spin coating process in the method shown in FIG. 2;
FIG. 4 shows a schematic perspective view of a cell culture membrane structure in accordance with an exemplary embodiment;
FIG. 5 shows a flowchart of a method for producing the cell culture membrane structure shown in FIG. 4;
FIG. 6 shows a schematic cross-sectional side view of a cell culture membrane structure in accordance with an exemplary embodiment;
FIG. 7 shows a schematic perspective view of a cell culture plate in accordance with an exemplary embodiment;
FIG. 8 shows a schematic cross-sectional view of one well and one cell culture membrane structure of the cell culture plate shown in FIG. 7, as taken along the line A-A of FIG. 7;
FIG. 9 shows a block-scheme of a cell culture apparatus in accordance with an exemplary embodiment;
FIGs. 10A-10E show different schematic views of a cell culture module included in the cell culture apparatus shown in FIG. 9 in accordance with exemplary embodiments, namely: FIG. 10A shows a schematic perspective view of the cell culture module, FIG. 10B shows a schematic side view of the cell culture module, FIG. 10C shows a schematic top view of the cell culture module, FIG. 10D shows a schematic side view of the cell culture module, and FIG. 10E shows a schematic side view of the cell culture module;
FIG. 11 shows a sectional view of one of two flow channels of the cell culture module, as taken within the area delimited by oval A in FIG. 10B;
FIG. 12 shows a schematic exploded view of a layered structure that represents one possible implementation example of the cell culture modules shown in FIGs. 10A-10E;
FIGs. 13A-13C show different schematic views of a cell culture module included in the cell culture apparatus in accordance with an exemplary embodiment. More specifically, FIG. 13A shows a schematic perspective view of the cell culture module, FIG. 13B shows a schematic side view of the cell culture module, and FIG. 13C shows a schematic top view of the cell culture module;
FIG. 14 shows a block diagram of a cell culture incubator in accordance with an exemplary embodiment;
FIG. 15 shows a flowchart of a method for cell cultivation by using the cell culture apparatus shown in FIG. 9 in accordance with an exemplary embodiment; and
FIG. 16 shows a flowchart of a method for cell cultivation by using the cell culture apparatus shown in FIG. 9 in accordance with an exemplary embodiment.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, the structure, devices and/or methods disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above" "below", "upper", "lower", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first side of a membrane discussed below could be called a second side of the membrane, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

As used in the embodiments disclosed herein, a culture medium, also referred to as a growth medium, may refer to a liquid, suspension, or gel designed to support cellular growth in an artificial environment, i.e., *in vitro.* There are different types of culture media suitable for growing different types of cells. In general, the culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the culture medium itself may comprise cells, bodies of cells (e.g., organoids and spheroids), lipid particles, including natural or engineered (e.g., exosomal microvesicles, vacuoles, micelles or lipid particles of various design, virus particles, nanoparticles, etc.).

The term "organoid" as used herein and hereafter refers to miniaturized structures of cells grown in 3D that, at least partially, resemble microanatomy of an organ in term of structure and function.

The term "spheroid" as used herein and hereafter refers to cell aggregates that are free-floating, and may be formed by a multicellular mixture of cells within a low-adhesion culture environment.

In one aspect is disclosed a cell culture membrane structure comprising:
- a membrane made of a membrane material, the membrane comprising:
- a first surface being partially deformed by one or more membrane structure cavities protruding from the first surface, and
- one or more through pores formed therein;
wherein the membrane material is selected based on at least one type of cells to be cultured on the membrane.

The cell culture membrane structures disclosed herein and hereafter may be used as a cell culture support adapted for specific cells, organoids, and/or spheroids to be cultured thereon. The membrane material is selected based on the at least one type of cells to be cultured on the membrane. By so doing, it is possible to alter different properties of the cell culture membrane structure (e.g., chemical and physical properties, such as hydrophilicity/hydrophobicity, stiffness, roughness, cell proliferation, attachment, mobility, survivability), thereby providing control over how a cell, organoid, and/or spheroid interacts with the membrane and other cells. Given this, the cell culture membrane structure may be adapted for certain one or more types of cells, organoids, and/or spheroids to be cultured thereon. Furthermore, the first surface being partially deformed by one or more membrane structure cavities protruding from the first surface may allow for increasing the areas of cells, organoids, and/or spheroids through the cavities. In addition, the one or more membrane structure cavities may provide a microenvironment for the cells, organoids, and/or spheroids to be cultured therein.

Additionally, or alternatively, each of the one or more membrane structure cavities has a cavity opening being each independently 50 - 3000 µm wide. In embodiments, each of the one or more membrane structure cavities has a cavity opening being each independently 50 - 3000 µm, 50 - 2000 µm, 50 - 1000 µm, 50 - 500 µm, 100 - 3000 µm, 200 - 3000 µm, 500 - 3000 µm, 1000 - 3000 µm, or 2000 - 3000 µm wide. By selecting the width of the cavity opening specific cells, organoids, and/or spheroids may be grown in the one or more membrane structure cavities. The cavity opening having a specific width may restrict or allow certain type of cells, organoids, and/or spheroids to be cultured in the one or more membrane structure cavities. For example, cells with a size (e.g., a width of the cell being 3500 µm) being larger than the width of the cavity opening (e.g., 100 µm) do not fit to enter a membrane structure cavity via the cavity opening.

It is to be understood that since the membrane comprises cavities protruding from the first surface of the membrane, each cavity opening of the one or more membrane structure cavities may be on a second surface of the membrane, i.e., each cavity opening and the second surface may be on the opposite side of the membrane in respect to the first surface, preferably each cavity opening and the second surface is on the opposite side of the membrane in respect to the first surface.

Additionally, or alternatively, each of the one or more membrane structure cavities has an inner width being in the range of 50 - 3000 µm. In embodiments, the one or more membrane structure cavities has one or more inner shapes each independently selected from sphere, cylinder, pyramid, and square. Additionally, or alternatively, the one or more membrane structure cavities has an inner shape selected from sphere, and the one or more membrane structure cavities has an inner radius being selected from the range of 50 - 3000 µm. By selecting the inner width or inner radius and/or the shape of the one or more membrane structure cavities specific cells, organoids, and/or spheroids may be grown in the one or more membrane structure cavities.

Additionally, or alternatively, the cell culture membrane structure further comprises at least one coating layer being made of a coating material, the at least one coating layer provided on the membrane such that the one or more membrane structure cavities and the one or more through pores of the membrane remain open, wherein the membrane material and the coating material are selected based on at least one type of cells to be cultured on the membrane and/or on the at least one coating layer. The coating material is selected based on the at least one type of cells, organoids, and/or spheroids to be cultured on the membrane and/or on the at least one coating layer, preferably on the at least one coating layer. By doing so, it is possible to further alter different properties of the cell culture membrane structure (e.g., chemical and physical properties, such as hydrophilicity/hydrophobicity, stiffness, roughness, cell proliferation, attachment, mobility, survivability), thereby providing control over how a cell, organoid, and/or spheroid interacts with the membrane and other cells. Given this, the cell culture membrane structure may be adapted for certain one or more types of cells, organoids, and/or spheroid to be cultured thereon.

Additionally, or alternatively, the at least one coating layer comprises one first coating layer provided on a second surface of the membrane. Additionally, or alternatively, the at least one coating layer comprises one first coating layer provided on a second surface of the membrane, the second surface being on the opposite side of the membrane in respect to the first surface. It is to be understood that the second surface may include, preferably includes, the inner surface of the one or more membrane structure cavities. One first coating layer provided on the second surface of the membrane may enhance the formation and/or growth of cells, organoids, and/or spheroids to be cultured on the one first coating layer. In particular, the first coating layer provided on the second surface of the membrane may enhance the formation and/or growth of cells, organoids, and/or spheroids to be cultured on the first coating layer in the one or more membrane structure cavities.

Additionally, or alternatively, the at least one coating layer comprises one first coating layer provided on the first surface of the membrane. It is to be understood that the first surface may be free of, preferably is free of, the inner surface of the one or more membrane structure cavities, and the first surface may be free of, preferably is free of, cavity openings. One first coating layer provided on the first surface of the membrane may enhance the formation and/or growth of cells, organoids, and/or spheroids to be cultured on the one first coating layer.

Additionally, or alternatively, the at least one coating layer comprises one first coating layer provided on a second surface of the membrane and one second coating layer provided on the first surface of the membrane, the second surface being on the opposite side of the membrane in respect to the first surface. Having one first coating layer provided on the second surface of the membrane and one second coating layer provided on the one first surface of the membrane may enhance the formation and/or growth of cells, organoids, and/or spheroids to be cultured on the one first coating layer and/or the one second coating layer. By coating the membrane on either side, it is possible to additionally alter different physical and chemical properties of the cell culture membrane structure. For example, it is possible to alter elastic properties of the cell culture membrane structure mainly in a vertical direction (i.e., a direction perpendicular to the membrane) by using the coatings on either side. In addition, cells, spheroids, and organoids of different types require specific biomimetic surface properties that may be achieved by the one first coating layer provided on the second surface of the membrane and one second coating layer provided on the one first surface of the membrane.

Additionally, or alternatively, the coating material of the at least one coating layer is the same or different for each of the first coating layer and the second coating layer. This may make it possible to study cell behavior on the cell culture membrane structure under different *in vitro* conditions.

Additionally, or alternatively, the coating material of the one first coating layer and the coating material of the one second coating layer are different. The formation and/or growth of cells, organoids, and/or spheroids of a first type may be enhanced on one of the one first coating layer and the one second coating layer while the formation and/or growth of cells, organoids, and/or spheroids of a second type may be enhanced on the another one of the one first coating layer and the one second coating layer. Alternatively, the formation and/or growth of cells, organoids, and/or spheroids of a first type may be enhanced on one of the one first coating layer and the one second coating layer while the formation and/or growth of cells, organoids, and/or spheroids of a second type may not be enhanced on the another one of the one first coating layer and the one second coating layer.

Additionally, or alternatively, the coating material of the one first coating layer and the coating material of the one second coating layer are the same. The formation and/or growth of cells, organoids, and/or spheroids may be enhanced on the one first coating layer in the one or more membrane structure cavities while the formation and/or growth of cells, organoids, and/or spheroids may have a reduced formation and/or growth of cells, organoids, and/or spheroids on the one second coating layer compared to the formation and/or growth of cells, organoids, and/or spheroids on the one first coating layer in the one or more membrane structure cavities.

Additionally, or alternatively, each of the first coating layer and the second coating layer has a different thickness and/or stiffness. In embodiments, each of the first coating layer and the second coating layer has a different thickness and stiffness. In embodiments, each of the first coating layer and the second coating layer has a different thickness or stiffness. In embodiments, the thickness of each of the first coating layer and the second coating layer may vary in the range 1 - 50 µm, 8 - 75 µm, 8 - 50 µm, 8 - 40 µm, 8 - 30 µm, 8 - 20 µm, 8 - 12 µm, 15 - 110 µm, 20 - 100 µm, 30 - 100 µm, 40 - 100 µm, 50 - 100 µm, 60 - 100 µm, 70 - 100 µm, 80 - 100 µm, or 90 - 100 µm. In embodiments, the thickness of each of the first coating layer and the second coating layer may vary in the range 1 - 50 µm. This may also make it possible to study the cell behavior on the cell culture membrane structure under different *in vitro* conditions.

Additionally, or alternatively, the thickness of the membrane is selected from the range 8 - 100 µm, 8 - 75 µm, 8 - 50 µm, 8 - 40 µm, 8 - 30 µm, 8 - 20 µm, 8 - 12 µm, 15 - 110 µm, 20 - 100 µm, 30 - 100 µm, 40 - 100 µm, 50 - 100 µm, 60- 100 µm, 70 - 100 µm, 80 - 100 µm, or 90 - 100 µm. In embodiments, the thickness of the membrane may vary in the range 8 - 12 µm, or 20 - 100 µm. The thicknesses of the membrane may affect the penetration and/or infiltration of molecules produced by cells, spheroids, or organoids from one side of the membrane to the other side (when cells, spheroids, or organoids are grown on the membrane of the cell culture membrane structure), and the thickness of the membrane may also effect on interactions between the cells, spheroids, or organoids being on both sides of the membrane.

Additionally, or alternatively, the at least one type of cells comprises a first type of cells to be cultured on the first coating layer and a second type of cells to be cultured on the second coating layer. This may allow one to study the behavior of different cells on the cell culture membrane structure.

Additionally, or alternatively, each of the through pores has a pore size each independently selected from a predefined range of values, and wherein the through pores are spaced from each other by a distance that is at least the predefined range of values. By varying the pore size within the predefined range of values and the distance between the pores being at least the predefined range of values, one may alter different physical and chemical properties of the membrane, thereby influencing the cell behavior on the cell culture membrane structure.

Additionally, or alternatively, the each of the through pores has a pore size each independently selected from a predefined range of values, and wherein the through pores are spaced from each other by a distance each independently selected from the predefined range of values, the predefined range of values being selected based on the at least one type of cells to be cultured on the membrane and/or on the at least one coating layer. In embodiments the predefined range of values is selected from 100 nm - 400 µm, 200 nm - 200 µm, 300 nm - 200 µm, 400 nm - 200 µm, 500 nm - 200 µm, 1 - 200 microns, 10 - 200 µm, 20 - 200 µm, 30 - 200 µm, and 35 - 200 µm, the predefined range of values being selected based on the at least one type of cells to be cultured on the membrane and/or on the at least one coating layer. In embodiments the predefined range of values is selected from 200 nm - 200 µm. By varying the pore size and the distance between the pores within the same range of values, one may alter different physical and chemical properties of the membrane, thereby influencing the cell behavior on the cell culture membrane structure.

Additionally, or alternatively, the predefined range of values is selected based on the at least one type of cells to be cultured on the at least one coating layer. This may also provide different physical and chemical properties of the membrane, thereby influencing the cell behavior on the cell culture membrane structure.

Additionally, or alternatively, the pore sizes of the through pores are selected to be less than cell sizes of the at least one type of cells to be cultured on the membrane and/or on the at least one coating layer. These embodiments are useful when it is required to prevent cells from migrating between opposite sides of the membrane through the pores.

Additionally, or alternatively, the at least one type of cells comprises a first type of cells and a second type of cells which are both to be cultured on one of the first coating layer and the second coating layer. This may allow one to study the behavior of different cells on the same side of the cell culture membrane structure.

Additionally, or alternatively, each of the through pores has a pore size each independently selected from a predefined range of values, the through pores being spaced from each other by a distance each independently selected from the predefined range of values, and wherein the predefined range of values is selected such that the through pores are configured to pass one of the first type of cells and the second type of cells therethrough and retain the other one of the first type of cells and the second type of cells. The predefined range of values is selected for example, but not limited to, based on sizes and/or elastic properties of cells of the both types. Each of such through pores may be spaced from each other by a distance varying within the same predefined range of values. By having such through pores in the membrane, it is possible to squeeze or filter cells of a certain type therethrough in situations when cells of two different types are initially grown on the first or second coating layers (i.e., on the same side of the membrane). After said filtering or squeezing, the membrane will have cells of one type on one side and cells of the another type on another (opposite) side. It is to be understood that when one or more through pores may have for example a circular cross-section shape, the pore size of the through pore may correspond to the diameter of the circular cross-section of the through pore.

Additionally, or alternatively, the membrane comprises a plurality of through pores formed therein, wherein the plurality of through pores formed therein are distributed in the membrane by one or more through pores groups comprising four through pores in each through pores group, wherein the four through pores of each through pores group being essentially evenly distributed in the through pores group. In embodiments, each of the through pores of each through pores group has a pore size of 200 ± 20 µm or of about 200 µm and the through pores being spaced from each other such that the four through pores form together an essentially square group of four through pores in each through pores group and adjacent through pores are spaced from each other by a distance of about 50 µm. It is to be understood that in these embodiments adjacent through pores are through pores on the same side of the square group having four equal sides and four equal angles being 90 degrees, the distance of the four essentially equal sides of each through pores group (being a square group) is 450 ± 40 µm or of about 450 µm, and the through pores not being adjacent through pores are through pores that are diagonally distributed in the essentially square group in respect to each other. Additionally, or alternatively, the cell culture membrane structure comprises a plurality of through pores, wherein the pore density of the cell culture membrane structure is 10³-10⁷ pores per millimeter square. It should be apparent that the pore density will depend on the pore size and spacing selected depending on particular applications.

Additionally, or alternatively, the membrane material is selected from the group consisting of polypropylene, polystyrene (PS), polydimethylsiloxane (PDMS), polycarbonate, polyethersulfone, polyethylene terephthalate, polyimide, polytetrafluoroethylene, silicone, and silicon nitride. By selecting from these membrane materials, one may also alter different physical and chemical properties of the cell culture membrane structure, thereby extending the range of its application.

Additionally, or alternatively, the coating material of the at least one coating layer is each independently selected from the group consisting of cell adherence promoters such as endothelial cells, and epithelial cells; extracellular matrices such as a fibronectin, and collagen; cell repellent compounds such as pluronic F127, biolipidure, and Teflon; hydrogels such as matrigel, cultrex, Fibrinogen, and Alginate; silicone, polyacrylamide, PEG polymer, acrylamide, gelatin, gelatin derivative such as gelatin methacrylates, and hyaluronics; cellulose, nanocellulose, and fibrin. It is to be understood that extracellular matrices may be cell adherence promoters. By selecting from these coating materials, one may also alter different physical and chemical properties of the cell culture membrane structure, thereby extending the range of its application.

Additionally, or alternatively, the coating material of the one first coating layer is cell adherence promoter or an extracellular matrix, and the one first coating layer form a cell monolayer on the second surface of the membrane. In embodiments, the coating material of the one first coating layer is endothelial cells, fibronectin, or collagen, and the one first coating layer form a cell monolayer on the second surface of the membrane.

Additionally, or alternatively, the coating material of the first coating layer and the second coating layer is each independently selected from cell adherence promoter and extracellular matrix, and the first coating layer and the second coating layer form a cell monolayer on the second surface and on the first surface, respectively. In embodiments, the coating material of the first coating layer and the second coating layer is endothelial cells, fibronectin, or collagen, and the first coating layer and the second coating layer form a cell monolayer on the second surface and on the first surface, respectively.

Additionally, or alternatively, the coating material of the first coating layer is selected from hydrogels such as collagen, fibronectin, fibrin, or gelatin. Using a coating material of the first coating layer selected from hydrogels such as collagen, fibronectin, fibrin, or gelatin may increase the physiological performance of cell culture membrane structure.

Additionally, or alternatively, the coating material of the first coating layer and the second coating layer is each independently selected from hydrogels such as collagen, fibronectin, fibrin, or gelatin. Using a coating material of the first coating layer and the second coating layer selected from hydrogels such as collagen, fibronectin, fibrin, or gelatin may increase the physiological performance of cell culture membrane structure.

Additionally, or alternatively, the coating material of the at least one coating layer is a selected from cell repellent compounds. In embodiments, the cell repellent compounds is selected from pluronic F127, biolipidure, and Teflon. By selecting from these coating materials, organoids may be grown inside each cavity that may allow for increasing the areas of vascularized organoids through the porous cavities.

Additionally, or alternatively, the coating material of the at least one coating layer is a hydrogel, such as collagen, fibronectin, fibrin, gelatin, or a gelatin derivative.

Additionally, or alternatively, the cell culture membrane structure further comprises at least one metal layer, carbon nanotube, nanosheet, graphene, or a nanomaterial of such nature, bonded onto the at least one coating layer.

Additionally, or alternatively, the cell culture membrane structure further comprises an organic compound (e.g., antifouling agents) and/or biomolecule (e.g., each independently selected from one or more from the group consisting of antigens, proteins, carbohydrates, antibody molecules) bonded onto the at least one coating layer. By using the organic compounds and/or biomolecules, it is possible to provide an even larger spectrum of functionality for the cell culture membrane structure.

Additionally, or alternatively, the cell culture membrane structure further comprises an insert wall attached to the cell culture membrane structure, an inner insert cavity, and an insert opening; wherein the inner insert cavity is surrounded by the insert opening, the membrane, and the insert wall; the insert wall extending from the membrane to the insert opening; and the insert cavity is located between the insert opening and the membrane. In these embodiments, the cell culture membrane structure may be used as an insert or compartment in various applications, since one may provide in the inner insert cavity of the cell culture membrane structure for example, but not limited to, culture medium comprising for example, but not limited to, cells, organoids, and/or spheroids. In embodiments, the insert wall comprises one or more walls. In embodiments, the insert wall is a bottomless hollow tube. In embodiments, the first surface of the membrane is located on the opposite side of the membrane in respect to the inner insert cavity. Additionally, or alternatively, the cavity opening of each of the one or more membrane structure cavity is on the same side of the membrane in respect to the inner insert cavity. In these embodiments, cells, organoids, and/or spheroids may be grown in the one or more membrane structure cavity from the inner insert cavity side of the cell culture membrane structure. In embodiments, the first surface of the membrane is located on the same side of the membrane in respect to the inner insert cavity. Additionally, or alternatively, the cavity opening of each of the one or more membrane structure cavity is on the opposite side of the membrane in respect to the inner insert cavity. In these embodiments, cells, organoids, and/or spheroids may be grown in the one or more membrane structure cavity located on the opposite side of the membrane in respect to the inner insert cavity side of the cell culture membrane structure.

Additionally, or alternatively, the membrane material is silicone, the average thickness of the membrane is in the range 20 - 100 µm, the membrane comprising the first surface being partially deformed by a plurality of membrane structure cavities protruding from the first surface; and a plurality of through pores formed therein, each of the plurality of membrane structure cavities has a cavity opening being each independently 50 - 3000 µm wide, each of the plurality of membrane structure cavities has an inner width being in the range of 50-3000 µm, the plurality of through pores of the membrane has an average pore size falling within a range from 20 µm to 200 µm, wherein the cell culture membrane structure further comprises at least one coating layer being made of a coating material, the at least one coating layer provided on the membrane such that the plurality of membrane structure cavities and the plurality of through pores of the membrane remain open, wherein the membrane material and the coating material are selected based on at least one type of cells to be cultured on the membrane and/or on the at least one coating layer, wherein the at least one coating layer comprises one first coating layer provided on a second surface of the membrane and one second coating layer provided on the first surface of the membrane, the second surface being on the opposite side of the membrane in respect to the first surface, the coating material of the one first coating layer and the one second coating layer are both a hydrogel, such as collagen, fibronectin, fibrin, gelatin, or a gelatin derivative; and the average thickness of each of the first coating layer and the second coating layer is in the range 1 - 50 µm, and the at least one type of cells to be cultured on the membrane and/or on the at least one coating layer comprises human brain vascular endothelial cells and human astrocytes. These cell culture membrane structures are beneficial, since the membrane made of silicone is flexible for brain vascular endothelial cells to be cultured efficiently in the cavities. The silicone membrane may act as a frame/scaffold for the hydrogel coating layers. The hydrogel layer may act as a porous media to allow liquid contact through cell culture membrane structure. Cells, organoids, and/or spheroids may be grown efficiently in the cavities. Using a hydrogel-based extracellular matrix as coating of the porous membrane will help increase the physiological performance of barrier models using the cell culture membrane structure. In addition, oxygen supply for the brain vascular endothelial cells and human astrocytes through the membrane and coating layers may be increased, being beneficial for the growth of the brain vascular endothelial cells and human astrocytes. Furthermore, using these cell culture membrane structures in cell culture apparatuses in methods for cell cultivation, it may allow for increasing the areas of vascularized organoids through the porous cavities and it may be possible to create artificial blood-brain barriers (BBBs) or create human mimicking artificial BBBs.

Additionally, or alternatively, the membrane material is polypropylene, polystyrene (PS), polydimethylsiloxane (PDMS), polycarbonate, polyethersulfone, polyethylene terephthalate, polyimide, or polytetrafluoroethylene, the average thickness of the membrane is in the range 8 - 12 µm, the membrane comprising the first surface being partially deformed by a plurality of membrane structure cavities protruding from the first surface, each of the plurality of membrane structure cavities has a cavity opening being each independently 50 - 3000 µm wide, each of the plurality of membrane structure cavities has an inner width being in the range of 50 - 3000 µm; and a plurality of through pores formed therein, the plurality of through pores of the membrane has an average pore size falling within a range from 0.2 µm to 10 µm, wherein the cell culture membrane structure further comprises at least one coating layer being made of a coating material, the at least one coating layer provided on the membrane such that the plurality of membrane structure cavities and the plurality of through pores of the membrane remain open, wherein the membrane material and the coating material are selected based on at least one type of cells to be cultured on the membrane and/or on the at least one coating layer, wherein the at least one coating layer comprises one first coating layer provided on a second surface of the membrane and one second coating layer provided on the first surface of the membrane, the second surface being on the opposite side of the membrane in respect to the first surface, the coating material of the one first coating layer and the one second coating layer is each independently selected from the group consisting of cell repellent compounds, such as pluronic F127, biolipidure, and Teflon; cell adherence promoter, or extracellular matrix; and the average thickness of each of the first coating layer and the second coating layer is in the range 1 - 50 µm, and the at least one type of cells to be cultured on the membrane and/or on the at least one coating layer comprises organoids, spheroids, or human brain vascular endothelial cells and human astrocytes. Cells, organoids, and/or spheroids may be grown efficiently in the cavities. These cell culture membrane structures are beneficial, since the membrane made of polypropylene, polystyrene (PS), polydimethylsiloxane (PDMS), polycarbonate, polyethersulfone, polyethylene terephthalate, polyimide, or polytetrafluoroethylene is rigid, and organoids may be grown efficiently in the cavities. When the membrane of the cell culture membrane structure is provided with one or more coating layers made of a coating material of a cell repellent compound, such as pluronic F127, biolipidure, and Teflon, cells, spheroids, and/or organoids inside each cavity may be formed when the cell culture membrane structure is used in cell cultivation. A cell culture membrane structure comprising a coating material of cell adherence promoter, or extracellular matrix, may form a cell monolayer on both sides of the membrane. These coatings of the porous membrane will help increase the physiological performance of barrier models. In addition, oxygen supply for the brain vascular endothelial cells and human astrocytes through the membrane and coating layers may be increased, being beneficial for the growth of the brain vascular endothelial cells and human astrocytes. Furthermore, using these cell culture membrane structures in the methods for cell cultivation, it may allow for increasing the areas of vascularized organoids through the porous cavities and it may be possible to create artificial blood-brain barriers (BBBs) or create human mimicking artificial BBBs.

In one aspect is provided a cell culture plate comprising:
a housing encompassing one or more wells, each well being arranged to retain a cell culture media, and
one or more cell culture membrane structures as defined in the present disclosure each being a replaceable insert configured to be immersed into the cell culture media such that each of the inner insert cavities of each of the replaceable inserts is in flow communication with one well of the housing via the through pores.

With such a cell culture plate, it is possible to grow cells of desired type(s) on a large amount of cell culture membrane structures simultaneously and under the same growing conditions.

In one aspect is provided a cell culture apparatus comprising:
one or more cell culture modules, each cell culture module of the one or more cell culture modules comprising:
   - at least two culture medium reservoirs each having a top part and a bottom part, the top part having an inlet for a culture medium and the bottom part having an outlet for the culture medium;
   - a first chamber configured to be arranged between the at least two culture medium reservoirs such that the first chamber and the at least two culture medium reservoirs are aligned with each other in a first direction, and
   - a cell culture membrane structure as defined in the present disclosure configured to be arranged between the first chamber and a second chamber such that the first chamber and the second chamber are in flow communication with each other via the through pores; or
   - a cell culture membrane structure as defined in the present disclosure configured to be arranged between the at least two culture medium reservoirs such that the cell culture membrane structure and the at least two culture medium reservoirs are aligned with each other in a first direction, the membrane of the cell culture membrane structure being arranged between the inner insert cavity and a second chamber such that the inner insert cavity and the second chamber are in flow communication with each other via the through pores;
wherein the second chamber being either arranged under and aligned with the first chamber, or arranged under and aligned with the cell culture membrane structure as defined in the present disclosure, in a second direction, the second direction having an angle being more than 0 degrees but 90 degrees or less in respect to the first direction, the second chamber having a bottom part having at least two lateral holes; and
   - at least two flow channels, each of the at least two flow channels being configured to pass a fluid or cell culture media therethrough, and each of the at least two flow channels connecting the outlet of the bottom part of one of the at least two culture medium reservoirs to one of the at least two lateral holes of the bottom part of the second chamber.

"A cell culture membrane structure as defined in the present disclosure configured to be arranged between the at least two culture medium reservoirs", "any of the cell culture membrane structure comprising an insert wall as disclosed in the present disclosure", and "cell culture membrane structure as defined in the present disclosure" as used herein and hereafter may refer to any cell culture membrane structure disclosed in the present disclosure further comprising an insert wall attached to the cell culture membrane structure, an inner insert cavity, and an insert opening; wherein the inner insert cavity is surrounded by the insert opening, the membrane, and the insert wall; the insert wall extending from the membrane to the insert opening; and the inner insert cavity is located between the insert opening and the membrane, the cell culture membrane structure configured to be arranged between the at least two culture medium reservoirs.

With this configuration, the cell culture apparatus may cultivate cells of the same or different types on the basal (i.e., bottom) surface of the porous membranes or on the apical (i.e., top) surface of the porous membrane in the cell culture modules under uniform and controlled *in vitro* conditions. Moreover, the deposition of the cells on the basal surface of the porous membranes may be provided without having to invert the apparatus or to use high concentrations of the cells in the culture medium to cause the cells to bond and cover the membrane. The cells deposited on the apical surface of the porous membrane may be of the same or other type compared to the cells deposited on the basal surface of the porous membrane. Thus, the first chamber thus configured may allow one to deposit a cell monolayer on the apical surface of the porous membrane and study the interaction of the cells deposited on the basal and apical surfaces of the membranes. Moreover, with such configuration, the first chamber is easier and cheaper to manufacture.

The at least one cell culture membrane structure is in contact with one of the at least one microfluidic flow channel. With such configuration, it is possible to use the microfluidic device for different biomimetic purposes, depending on the cells to be cultured on the cell culture membrane structure(s).

The terms "second direction having an angle being more than 0 degrees but 90 degrees or less in respect to the first direction" as disclosed herein and hereafter refers to a second direction that may be perpendicular to the first direction, or the angle between the second direction and the first direction is more than 0 degrees but less than 90 degrees. In embodiments, the second direction is perpendicular to the first direction.

The term "cell culture apparatus" as disclosed in the present disclosure may refer to an apparatus having various wells (e.g., reservoirs, chambers, etc.) connected by microchannels in which fluids (e.g., culture media) will exhibit microfluidic behavior in their flow through the microchannels. Such a cell culture apparatus is also referred to as a microfluidic chip in this technical field. The microfluidic cell culture performed by the cell culture apparatus is generally related to cell culture, maintenance and perturbation in micro-scale fluid volumes. The reasons behind the popularity of the microfluidic cell culture are both economic and scientific. The microfluidic chips have the advantages of *in vitro* cell culture (high-throughput, parallel experiments, experiments may be done at the discretion of an experimenter, no need for specialized infrastructure and personnel, etc.) with *in vivo* like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

In the embodiments disclosed herein, each microchannel of the cell culture apparatus is also referred to as a flow channel and may relate to a sub-millimeter-scale channel that has a hydraulic diameter below 1 mm and is used for fluidically connecting the wells of the cell culture apparatus. In other words, the microchannel or flow channel generally denotes a channel configured to pass different fluids, such, for example, as culture media (e.g., cell suspensions), reagents, or gels, in micro-scale volumes. The microchannel may be shaped such that it has appropriate flow characteristics (e.g., flow rates) depending on particular applications. For example, the microchannel may have a rectangular, e.g., square, or rounded cross-section, as well as be straight, bended, or tilted towards a required direction.

The microfluidic cell culture may involve using a porous membrane sandwiched between two flow layers within a microchannel or between a culture chamber and a microchannel for cell deposition. However, the membrane-based cell culture apparatuses known so far suffer from low throughput and incompatibility with available liquid handling systems (e.g., microtiter plate formats) and imaging systems. Furthermore, the flow control in such apparatuses is often limited to one method and not flexible enough to adapt to different resource settings. On top of that, the cell deposition on the basal (i.e., bottom) surface of the porous membrane(s) is possible in the current cell culture apparatuses only with very high cell concentrations, liquid flow control, and by inverting the cell culture apparatus.

The exemplary embodiments disclosed herein provide a technical solution that allows mitigating or even eliminating the drawbacks of the prior art. In particular, the technical solution disclosed herein provides a cell culture apparatus comprising one or more cell culture modules.

Additionally, or alternatively, the first surface of the membrane is located on the opposite side or the same side of the membrane in respect to the first chamber of the cell culture membrane structure, or the first surface of the membrane is located on the opposite side or the same side of the membrane in respect to the inner insert cavity of the cell culture membrane structure as defined in the present disclosure.

Additionally, or alternatively, the cell culture membrane structure as defined in the present disclosure is any cell culture membrane structure disclosed in the present disclosure that further comprises an insert wall attached to the cell culture membrane structure, an inner insert cavity, and an insert opening; wherein the inner insert cavity is surrounded by the insert opening, the membrane, and the insert wall; the insert wall extending from the membrane to the insert opening; and the insert cavity is located between the insert opening and the membrane, wherein the cell culture membrane structure being configured to be arranged between the at least two culture medium reservoirs such that the cell culture membrane structure and the at least two culture medium reservoirs are aligned with each other in a first direction, the membrane of the cell culture membrane structure being arranged between the inner insert cavity and a second chamber such that the inner insert cavity and the second chamber are in flow communication with each other via the through pores. Such cell culture apparatuses are beneficial, since the cell culture membrane structure may be removably arranged in the cell culture apparatus, i.e., the cell culture membrane structure may be attached, e.g., using a snap-fit, to the cell culture apparatus when the cell culture apparatus is in use, or the cell culture membrane structure may be removed from the cell culture apparatus when the cell culture apparatus is not in use.

Additionally, or alternatively, the insert wall comprises one or more walls. Additionally, or alternatively, the insert wall is a bottomless hollow tube.

Additionally, or alternatively, the cell culture membrane structure of the cell culture apparatus is any cell culture membrane structure disclosed in the present disclosure that is free of an insert wall attached to the cell culture membrane structure, in combination with a first chamber configured to be arranged between the at least two culture medium reservoirs such that the first chamber and the at least two culture medium reservoirs are aligned with each other in a first direction. Such cell culture apparatuses are beneficial, since the first chamber and the cell culture membrane structure may be removably arranged in the cell culture apparatus, i.e., the first chamber and the cell culture membrane structure may be attached, e.g., using one or more snap-fits, to the cell culture apparatus when the cell culture apparatus is in use or the first chamber and the cell culture membrane structure may be removed from the cell culture apparatus when the cell culture apparatus is not in use.

Additionally, or alternatively, the apparatus comprises the cell culture membrane structure as defined in the present disclosure being any cell culture membrane structure disclosed in the present disclosure that further comprises an insert wall attached to the cell culture membrane structure, an inner insert cavity, and an insert opening; wherein the inner insert cavity is surrounded by the insert opening, the membrane, and the insert wall; the insert wall extending from the membrane to the insert opening; and the insert cavity is located between the insert opening and the membrane, wherein the apparatus further comprises a first chamber configured to be arranged between the at least two culture medium reservoirs such that the first chamber and the at least two culture medium reservoirs are aligned with each other in the first direction, wherein the cell culture membrane structure being configured to be detachably inserted in the first chamber, the second chamber being arranged under and aligned with the cell culture membrane structure in the second direction. Such cell culture apparatuses are beneficial, since the first chamber and the cell culture membrane structure may be removably arranged in the cell culture apparatus, i.e., the cell culture membrane structure may be removably inserted in the first chamber that may be removably arranged in the cell culture apparatus, e.g., using one or more snap-fits, when the cell culture apparatus is in use or the first chamber and the cell culture membrane structure may be removed from the cell culture apparatus when the cell culture apparatus is not in use.

Additionally, or alternatively, the apparatus further comprises a flow driving unit configured to cause the culture medium to flow, in each cell culture module of the one or more cell culture modules, between the at least two culture medium reservoirs via the at least two flow channels and the second chamber. With this configuration, the cell culture apparatus may cultivate cells of the same or different types on the basal (i.e., bottom) surface of the porous membranes in the cell culture modules under uniform and controlled *in vitro* conditions. Moreover, the deposition of the cells on the basal surface of the porous membranes may be provided without having to invert the apparatus or to use high concentrations of the cells in the culture medium to cause the cells to bond and cover the membrane. It is to be understood that due, to the one or more through pores, the culture medium may flow, in each cell culture module of the one or more cell culture modules, also to the first chamber or the inner insert cavity of the cell culture membrane structure as defined in the present disclosure configured to be arranged between the at least two culture medium reservoirs via the at least two flow channels and the second chamber.

Additionally, or alternatively, the apparatus comprises a plurality of cell culture modules arranged adjacent to each other. With this configuration, the cell culture apparatus may cultivate cells more effectively.

Additionally, or alternatively, the first chamber in at least one cell culture module of the one or more cell culture modules is implemented as a bottomless hollow tube. With this first chamber, it is possible to deposit cells on the apical (i.e., top) surface of the porous membrane. The cells deposited on the apical surface of the porous membrane may be of the same or other type compared to the cells deposited on the basal surface of the porous membrane. Thus, the first chamber thus configured may allow one to deposit a cell monolayer on the apical surface of the porous membrane and study the interaction of the cells deposited on the basal and apical surfaces of the membranes. Moreover, with such configuration, the first chamber is easier and cheaper to manufacture.

Additionally, or alternatively, the first chamber in at least one cell culture module of the one or more cell culture modules is implemented as a hollow tube having a curved or angled bottom, the curved or angled bottom having at least one first chamber cavity and at least one first chamber hole formed in each of the at least one first chamber cavity. With this first chamber, it is possible to deposit cells on separate portions of the apical surface of the porous membrane. This configuration of the first chamber is especially useful for forming similar or different particles under study (e.g., organoids or spheroids) on the apical surface of the porous membrane and study their interaction with the cells deposited on the basal surface of the porous membrane.

Additionally, or alternatively, the curved or angled bottom is coated, from outside, with a cell adhesion enhancing layer such that the at least one first chamber hole of the at least one first chamber cavity remains open, the cell adhesion enhancing layer being made of a hydrophilic material, such as type I collagen. With such a coating layer, the cells may clump outside the bottom of the first chamber and further adhere onto the apical surface of the porous membrane. Thus, this coating layer allows for less cells to be used in an experiment and more precise deposition of the cells on the apical surface of the porous membrane. In turn, the smaller number of cells also means less cell death or, in other words, more healthy cells in the cell culture as dead cells tend to release factors promoting apoptosis.

Additionally, or alternatively, the second chamber has a height falling within a range selected from 50 - 150 µm. By using such a second chamber, it is possible to make the cell culture apparatus as disclosed in the present disclosure more compact.

Additionally, or alternatively, the second chamber in at least one cell culture module of the one or more cell culture modules is coated, from inside, with a cell adhesion enhancing layer, the cell adhesion enhancing layer being made of a hydrophilic material, such as type I collagen. With such a coating layer, the cells may attach onto the basal surface of the porous membrane more efficiently. Thus, this coating layer allows for less cells to be used in an experiment and more precise deposition of the cells on the basal surface of the porous membrane. In turn, the smaller number of cells also means less cell death or, in other words, more healthy cells in the cell culture as dead cells tend to release factors promoting apoptosis.

Additionally, or alternatively, each of the at least two flow channels in at least one cell culture module of the one or more cell culture modules is coated, from inside, with a cell adhesion enhancing layer, the cell adhesion enhancing layer being made of a hydrophilic material, such as type I collagen. By using the adhesion enhancing layer, cell attachment onto the sides of the flow channels (as well as on the sides of the second chamber and the porous membrane) becomes stronger, cell proliferation is appropriate to a tissue being modelled, and cell viability is better. Selecting the correct adhesion enhancing layer also plays a role in the proper communication between cell types. Thus, this coating layer allows for less cells to be used in an experiment and more precise deposition of the cells on the basal surface of the porous membrane. In turn, the smaller number of cells also means less cell death or, in other words, more healthy cells in the cell culture as dead cells tend to release factors promoting apoptosis.

Additionally, or alternatively, the flow driving unit is configured to cause the culture medium to flow, in each cell culture module of the one or more cell culture modules, between the at least two culture medium reservoirs by:
- supplying a compressed gas or a pressurized air to the inlet of each of the at least two culture medium reservoirs; or
- pipetting the culture medium from one of the at least two culture medium reservoirs to another of the at least two culture medium reservoirs at regular time intervals or based on a level of the culture medium in each of the at least two culture medium reservoirs; or
- periodically rocking the one or more cell culture modules.

Thus, unlike current microfluidic cell culture devices, the cell culture apparatus as disclosed in the present disclosure is compatible with different flow control means, i.e., pneumatic pump-actuated flow control, and pumpless flow control (which is provided by means of gravity as a result of rocking the plurality of cell culture modules or by means of culture medium pipetting).

Additionally, or alternatively, each of the at least two culture medium reservoirs in at least one cell culture module of the one or more cell culture modules is implemented as a hollow tube. With such configuration, the culture medium reservoirs are easier and cheaper to manufacture.

Additionally, or alternatively, the bottom part of each of the at least two culture medium reservoirs has a positively tapered profile. By using such a configuration of the culture medium reservoirs, it is possible to cause the whole culture medium contained in the culture medium reservoirs to flow to the bottom towards the second chamber even when the apparatus is tilted, so that no culture medium is left in the culture medium reservoirs. This also helps to reduce the number of cells that is needed to be deposited or grown on the basal surface of the porous membrane. Additionally, this configuration of the culture medium reservoirs may allow one to achieve optimal flow rates in each cell culture module.

Additionally, or alternatively, at least one cell culture module of the one or more cell culture modules further comprises at least two electrodes, wherein each of the at least two electrodes is independently arranged in the first chamber, the cell culture membrane structure (600) as defined in claim 5, the second chamber, in one or more of the at least two culture medium reservoirs, or the membrane, or any combinations thereof. Additionally, or alternatively, at least one cell culture module of the one or more cell culture modules further comprises a first electrode arranged in the first chamber or any of the cell culture membrane structure comprising an insert wall as disclosed in the present disclosure, and a second electrode arranged in the second chamber. These electrodes may serve different purposes. For example, they may be used to feed electrical pulses to muscle or nerve cells present in the first chambers, the cell culture membrane structures and second chambers or be coupled to other sensors of various types. More specifically, these electrodes may be used to perform real-time Transepithelial/Transendothelial Electrical Resistance (TEER) measurements; in this case, signals may be read out by an external control unit.

Additionally, or alternatively, at least one cell culture module of the one or more cell culture modules further comprises a first electrode arranged in one or more of the at least two culture medium reservoirs and a second electrode arranged in the first chamber or any of the cell culture membrane structure comprising an insert wall as disclosed in the present disclosure. This arrangement of the first and second electrodes may be also used to perform the TEER measurements, for example.

Additionally, or alternatively, at least one cell culture module of the one or more cell culture modules further comprises at least two third electrodes arranged in the first chamber or any of the cell culture membrane structure comprising an insert wall as disclosed in the present disclosure. The third electrodes may be arranged on the same wall or on opposite walls inside the first chamber, or they may be arranged on the same wall or on opposite walls inside any of the cell culture membrane structure comprising an insert wall as disclosed in the present disclosure. These electrodes may be used to perform liquid/medium level measurements in the first chamber or in any of the cell culture membrane structure comprising an insert wall as disclosed in the present disclosure.

Additionally, or alternatively, at least one cell culture module of the one or more cell culture modules further comprises at least two fourth electrodes imbedded into the membrane. These electrodes may be used, for example, to provide different stimulus signals to the cells deposited on the apical and/or basal surface of the membrane.

Additionally, or alternatively, at least one cell culture module of the one or more cell culture modules further comprises at least one of an oxygen sensor, a pH sensor and a CO₂ sensor in the first chamber, any of the cell culture membrane structure comprising an insert wall as disclosed in the present disclosure, or the second chamber. These sensors enable real-time measurements of parameters crucial for cell behavior.

Additionally, or alternatively, the bottom part of the second chamber being arranged higher than the bottom part of each of the at least two culture medium reservoirs. The basal chamber may have a bottom part arranged higher than a bottom part of each of the culture medium reservoirs. In this apparatus configuration, flows of culture media may be perfused and regulated between the culture medium reservoirs in each cell culture module by either placing the apparatus on a rocking platform or connecting the apparatus to a pneumatic pump. Moreover, the apparatus thus configured may cultivate cells of the same or different types on the basal surface of the porous membranes in the cell culture modules under uniform and controlled *in vitro* conditions. The deposition of the cells on the basal surface of the porous membranes may be provided without having to invert the apparatus and to use high concentrations of the cells in the culture medium.

Additionally, or alternatively, each of the at least two flow channels extends at least partly at a tilting angle to the first direction, the tilting angle falling within a range of 5 degrees to 45 degrees. With such titled flow channels, it is possible to achieve appropriate flow characteristics and, therefore, improve cell delivery to the second chamber.

Additionally, or alternatively, the one or more through pores of the membrane has an average pore size falling within a range from 0.2 µm to 200 µm. In embodiments, the one or more through pores of the membrane has an average pore size falling within a range from 200 nm - 200 µm, 300 nm - 200 µm, 400 nm - 200 µm, 500 nm - 200 µm, 1 - 200 µm, 10 - 200 µm, 20 - 200 µm, 30 - 200 µm, 35 - 200 µm, and 0.2 - 10 µm. By varying the average pore size within this range, it is possible to study the behavior of cells of different sizes, which are deposited on one or each of the apical and basal surfaces of the membrane.

Additionally, or alternatively, each of the at least two flow channels in at least one cell culture module of the one or more cell culture modules has a variable channel height and/or a variable channel width which increase towards the second chamber. In embodiments, each of the at least two flow channels in at least one cell culture module of the one or more cell culture modules has a variable channel height or a variable channel width which increase towards the second chamber. In embodiments, each of the at least two flow channels in at least one cell culture module of the one or more cell culture modules has a variable channel height and a variable channel width which increase towards the second chamber. With this configuration of the flow channels, it is possible to improve flow characteristics in each cell culture module, thereby improving the cell delivery to the second chamber and, consequently, the cell attachment to the basal surface of the porous membrane. In embodiments, the variable channel width gradually increases towards the second chamber. This may allow achieving better retainment of the laminar flow of the culture medium in the flow channels.

Additionally, or alternatively, each of the at least two flow channels in at least one cell culture module of the one or more cell culture modules comprises a first channel portion and a second channel portion, the first channel portion extending from the outlet of the bottom part of one of the at least two culture medium reservoirs and being parallel to the first direction, the second channel portion connecting the first channel portion to one of the at least two lateral holes of the second chamber, the second channel portion being tilted relative to the first direction. With this configuration of the flow channels, it is possible to improve flow characteristics in each cell culture module, thereby improving the cell delivery to the second chamber.

In one aspect is provided a cell culture incubator comprising:
the cell culture apparatus as disclosed in the present disclosure; and
a pipetting station configured to feed the culture medium to each of the at least two culture medium reservoirs in each cell culture module of the one or more cell culture modules. With this configuration, the cell culture incubator may cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

In one aspect is provided a method for producing a cell culture membrane structure, comprising:
- providing a membrane made of a membrane material;
- forming one or more through pores in the membrane;
- deforming the membrane comprising one or more through pores formed therein, thereby obtaining one or more membrane structure cavities protruding from a first surface of the membrane;
wherein the membrane material is selected based on at least one type of cells to be cultured on the membrane. By altering the membrane materials depending on cell types, it is possible to alter different properties of the cell culture membrane structure (e.g., chemical and physical properties, such as hydrophilicity/hydrophobicity, stiffness, roughness, cell proliferation, attachment, mobility, survivability, etc.), thereby providing control over how a cell interacts with the membrane and other cells. Given this, the cell culture membrane structure may be adapted for certain one or more types of cells to be cultured thereon.

Additionally, or alternatively, the step of forming one or more through pores in the membrane is performed by using at least one of particle/fluid bombardment, etching and laser burning. These techniques may allow one to form the through pores in the membrane more efficiently.

Additionally, or alternatively, the deforming the membrane comprising one or more through pores formed therein is performed by using vacuum thermal forming process. In embodiments, the membrane material of the membrane used in the vacuum thermal forming process is selected from polypropylene, polystyrene (PS), polydimethylsiloxane (PDMS), polycarbonate, polyethersulfone, polyethylene terephthalate, polyimide, and polytetrafluoroethylene. These techniques may allow one to form the through pores in the membrane more efficiently.

Additionally, or alternatively, the method further comprising:
- applying at least one coating layer on the membrane such that the one or more membrane structure cavities and the one or more through pores of the membrane remain open, the at least one coating layer being made of a coating material;
wherein the membrane material and the coating material are selected based on at least one type of cells to be cultured on the membrane and/or on the at least one coating layer. By altering the coating materials depending on cell types, it is possible to alter different properties of the cell culture membrane structure (e.g., chemical and physical properties, such as hydrophilicity/hydrophobicity, stiffness, roughness, cell proliferation, attachment, mobility, survivability, etc.), thereby providing control over how a cell interacts with the membrane and other cells. Given this, the cell culture membrane structure may be adapted for certain one or more types of cells to be cultured thereon.

Additionally, or alternatively, the applying at least one coating layer on the membrane comprises: applying the at least one coating layer on the membrane in a continuous manner; and clearing the through pores of the membrane by using etching and/or air or liquid pressurizing techniques. By so doing, it is possible to remove the coating material from the pores more efficiently.

Additionally, or alternatively, the applying at least one coating layer on the membrane is performed by using one of spin coating, blade coating, spray coating, immersion coating, brush coating, stamping, and electrospraying. These techniques may allow one to apply the coating layer(s) on the membrane more efficiently.

An alternative method for producing a cell culture membrane structure is provided, the method comprising:
- providing a membrane material of silicone;
- performing a liquid silicone injection molding process using the membrane material of silicone, thereby obtaining a cell culture membrane structure comprising one or more through pores in the membrane and one or more membrane structure cavities protruding from a first surface of the membrane. This method may allow one to produce a cell culture membrane structure comprising through pores in the membrane, wherein the membrane comprises a first surface being partially deformed by one or more membrane structure cavities protruding from the first surface, more efficiently.

In one aspect is provided a method for producing a cell culture membrane structure, comprising:
- providing a membrane made of a membrane material,
- applying at least one coating layer on the membrane in a continuous manner, the at least one coating layer being made of a coating material; and
- forming through pores coming through the at least one coating layer and the membrane;
wherein the membrane material and the coating material are selected based on at least one type of cells to be cultured on the at least one coating layer. By altering the membrane and coating materials depending on cell types, it is possible to alter different properties of the cell culture structure (e.g., chemical and physical properties, such as hydrophilicity/hydrophobicity, stiffness, roughness, cell proliferation, attachment, mobility, survivability, etc.), thereby providing control over how a cell interacts with the membrane and other cells. Given this, the cell culture membrane structure may be adapted for certain one or more types of cells to be cultured thereon.

Additionally, or alternatively, the membrane made of a membrane material comprises a first surface being partially deformed by one or more membrane structure cavities protruding from the first surface.

Additionally, or alternatively, the method further comprises, before providing a membrane made of a membrane material, providing a membrane material of polydimethylsiloxane (PDMS), performing soft-lithography or liquid silicone injection molding using the PDMS for preparing a membrane made of PDMS comprising a first surface being partially deformed by one or more membrane structure cavities protruding from the first surface, thereby membrane made of PDMS comprising a first surface being partially deformed by one or more membrane structure cavities protruding from the first surface. These techniques may allow one to form the one or more membrane structure cavities in the membrane more efficiently.

Additionally, or alternatively, the method further comprises, before applying at least one coating layer, before forming through pores, or after forming through pores, deforming the membrane, thereby obtaining one or more membrane structure cavities protruding from a first surface of the membrane. The deforming the membrane is performed such that one or more membrane structure cavities protruding from a first surface of the membrane is obtained. The cell culture membrane structure may be adapted for certain one or more types of cells to be cultured thereon, in addition in the one or more membrane structure cavities.

Additionally, or alternatively, the applying at least one coating layer on the membrane in a continuous manner is performed by using one of spin coating, blade coating, spray coating, immersion coating, brush coating, stamping, and electrospraying. By using these techniques, it is possible to apply the coating layer(s) on the membrane more efficiently.

Additionally, or alternatively, the forming through pores coming through the at least one coating layer and the membrane is performed by using at least one of particle/fluid bombardment, etching and laser burning. These techniques may allow one to form the through pores in the whole cell culture membrane structure more efficiently.

Additionally, or alternatively, the deforming the membrane is performed by using vacuum thermal forming process. In embodiments, the membrane material of the membrane used in the vacuum thermal forming process is selected from polypropylene, polystyrene (PS), polydimethylsiloxane (PDMS), polycarbonate, polyethersulfone, polyethylene terephthalate, polyimide, and polytetrafluoroethylene. These techniques may allow one to form the one or more membrane structure cavities protruding from the first surface of the membrane in the membrane more efficiently.

In one aspect is provided a method for cell cultivation by using the cell culture apparatus as disclosed in the present disclosure, the method comprising:
(a) providing the culture medium to one of the at least two culture medium reservoirs in each cell culture module of the one or more cell culture modules; and
(b) causing the culture medium to flow from said one of the at least two culture medium reservoirs to the rest of the at least two culture medium reservoirs in each cell culture module of the one or more cell culture modules for a predefined time period, the predefined time period being selected based on the culture medium. By so doing, it is possible to cultivate cells of the same or different types on the porous membranes of the cell culture modules under unform and controlled in vitro conditions. In addition, cells may be cultivated more efficiently since the one or more cavities of the membrane provide increased area of cells, organoids, and/or spheroids through the porous cavities. Moreover, the deposition of the cells, organoids, and/or spheroids on the basal surface of the porous membranes may be provided without having to invert the cell culture apparatus or to use high concentrations of the cells in the culture medium to cause the cells to bond and cover the membrane.

Additionally, or alternatively, wherein, in (b), the causing the culture medium to flow is caused by a flow driving unit. By so doing, it is possible to cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

Additionally, or alternatively, the method further comprises, during (b), feeding another culture medium via the first chamber or any of the cell culture membrane structure comprising an insert wall as disclosed in the present disclosure towards the membrane in at least one cell culture module of the one or more cell culture modules. By so doing, it is possible to cultivate a first type of cells on the basal surface of the porous membranes and a different second type of cells on the apical surface of the porous membranes, thereby generating different cell co-cultures. In addition, cells may be cultivated more efficiently since the one or more cavities of the membrane provide increased area of cells, organoids, and/or spheroids through the porous cavities. Moreover, the deposition of the cells, organoids, and/or spheroids on the basal surface and/or on the apical surface of the porous membranes may be provided without having to invert the cell culture apparatus or to use high concentrations of the cells in the culture medium to cause the cells to bond and cover the membrane.

Additionally, or alternatively, the method further comprises, before (a), feeding another culture medium via the first chamber or any of the cell culture membrane structure comprising an insert wall as disclosed in the present disclosure towards the membrane in at least one cell culture module of the one or more cell culture modules.

Additionally, or alternatively, the method further comprises, after (b), incubating the cell culture apparatus for a predefined time period, the predefined time period being selected based on the culture medium and/or the another culture medium.

Additionally, or alternatively, wherein (b) comprises applying a positive pressure to the at least two culture medium reservoirs in each cell culture module of the one or more cell culture modules for the predefined time period. By so doing, it is possible to provide a proper culture medium flow in each cell culture module, thereby improving cell cultivation on the porous membranes in each cell culture module.

Additionally, or alternatively, wherein (b) comprises applying a positive pressure to said one of the at least two culture medium reservoirs in each cell culture module of the one or more cell culture modules for the predefined time period, while capping the rest of the at least two culture medium reservoirs. By so doing, it is possible to provide a proper culture medium flow in each cell culture module, thereby improving cell cultivation on the porous membranes in each cell culture module.

Additionally, or alternatively, wherein the at least two culture medium reservoirs in each cell culture module of the one or more cell culture modules comprises a first culture medium reservoir and a second culture medium reservoir, and wherein (b) comprises:
- applying a first positive pressure to the first culture medium reservoir in each cell culture module of the one or more cell culture modules for the predefined time interval, while capping the second culture medium reservoir; and
- applying a second positive pressure to the second culture medium reservoir in each cell culture module of the one or more cell culture modules for the predefined time interval, while capping the first culture medium reservoir. By so doing, it is possible to provide a proper culture medium flow in each cell culture module, thereby improving cell cultivation on the porous membranes in each cell culture module.

In one aspect is provided a method for cell cultivation by using the cell culture apparatus as disclosed in the present disclosure, the method comprising:
(a) providing the culture medium to one or more of the at least two culture medium reservoirs in each cell culture module of the one or more cell culture modules;
(b) causing the culture medium to flow to the second chamber from said one or more of the at least two culture medium reservoirs in each cell culture module of the one or more cell culture modules;
(c) placing the cell culture apparatus in an inverted position; and
(d) incubating the cell culture apparatus in the inverted position for a predefined time period, the predefined time period being selected based on the culture medium. By so doing, it is possible to cultivate cells of the same or different types on the porous membranes of the cell culture modules under unform and controlled *in vitro* conditions. Although the deposition of the cells on the basal surface of the porous membranes is provided by inverting the cell culture apparatus, the method provided does not require high concentrations of the cells to be used in the culture medium to cause the cells to bond and cover the membrane.

Additionally, or alternatively, wherein, in (b), the causing the culture medium to flow is caused by a flow driving unit. By so doing, it is possible to cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

Additionally, or alternatively, the method further comprising, during (b), feeding another culture medium via the first chamber or any of the cell culture membrane structure comprising an insert wall as disclosed in the present disclosure towards the membrane in at least one cell culture module of the one or more cell culture modules. By so doing, it is possible to cultivate a first type of cells on the basal surface of the porous membranes and a different second type of cells on the apical surface of the porous membranes, thereby generating different cell co-cultures. In addition, cells may be cultivated more efficiently since the one or more cavities of the membrane provide increased area of cells, organoids, and/or spheroids through the porous cavities. Moreover, the deposition of the cells, organoids, and/or spheroids on the basal surface and/or on the apical surface of the porous membranes may be provided without having to use high concentrations of the cells in the culture medium to cause the cells to bond and cover the membrane.

Additionally, or alternatively, the method further comprises, before (a), feeding another culture medium via the first chamber or any of the cell culture membrane structure comprising an insert wall as disclosed in the present disclosure towards the membrane in at least one cell culture module of the one or more cell culture modules.

Additionally, or alternatively, the culture medium comprises brain vascular endothelial cells and said another culture medium comprises astrocytes. In embodiments, the brain vascular human astrocytes are human brain vascular endothelial cells. In embodiments, the astrocytes are human astrocytes. By using these types of cells, it is possible to create artificial blood-brain barriers (BBBs) or create human mimicking artificial BBBs.

Additionally, or alternatively, the culture medium comprises astrocytes and said another culture medium comprises brain vascular endothelial cells. In embodiments, the brain vascular human astrocytes are human brain vascular endothelial cells. In embodiments, the astrocytes are human astrocytes. By using these types of cells, it is possible to create artificial blood-brain barriers (BBBs) or create human mimicking artificial BBBs.

Additionally, or alternatively, the cell culture membrane structure comprising the membrane made of silicone, the average thickness of the membrane is in the range 20 - 100 µm, the membrane comprising the first surface being partially deformed by a plurality of membrane structure cavities protruding from the first surface; and a plurality of through pores formed therein, each of the plurality of membrane structure cavities has a cavity opening being each independently 50 - 3000 µm wide, each of the plurality of membrane structure cavities has an inner width being in the range of 50 - 3000 µm, the plurality of through pores of the membrane has an average pore size falling within a range from 20 µm to 200 µm, wherein the cell culture membrane structure further comprises at least one coating layer being made of a coating material, the at least one coating layer provided on the membrane such that the plurality of membrane structure cavities and the plurality of through pores of the membrane remain open, wherein the membrane material and the coating material are selected based on at least one type of cells to be cultured on the membrane and/or on the at least one coating layer, wherein the at least one coating layer comprises one first coating layer provided on a second surface of the membrane and one second coating layer provided on the first surface of the membrane, the second surface being on the opposite side of the membrane in respect to the first surface, the coating material of the one first coating layer and the one second coating layer are both a hydrogel, such as collagen, fibronectin, fibrin, gelatin, or a gelatin derivative; and the average thickness of each of the first coating layer and the second coating layer is in the range 1 - 50 µm, and the culture medium comprises human brain vascular endothelial cells and said another culture medium comprises human astrocytes. Alternatively, the culture medium comprises human astrocytes and said another culture medium comprises human brain vascular endothelial cells. It is to be understood that depending on which side of the membrane the membrane structure cavities are protruding from and hence, on which side of the membrane the cavity openings are (i.e., in the second chamber side of the membrane or on the opposite side of the membrane in respect of the second chamber), either the culture medium or the another culture medium comprises the human brain vascular endothelial cells and, therefore, either the another culture medium or the culture medium comprises the human astrocytes. It is to be understood that the at least one type of cells (to be cultured on the membrane and/or on the at least one coating layer) comprises one first type of cells being the human brain vascular endothelial cells; and one second type of cells being the human astrocytes. These methods are beneficial, since the membrane made of silicone is flexible for brain vascular endothelial cells to be cultured efficiently in the cavities. The silicone membrane may act as a frame/scaffold for the hydrogel coating layers. The hydrogel layer may act as a porous media to allow liquid contact between the first chamber or any of the cell culture membrane structure comprising an insert wall as disclosed in the present disclosure and the second chamber of the microfluidic cell culture apparatus. Using a hydrogel-based extracellular matrix as coating of the porous membrane will help increase the physiological performance of the barrier models. In addition, oxygen supply for the brain vascular endothelial cells and human astrocytes through the membrane and coating layers are increase, being beneficial for the growth of the brain vascular endothelial cells and human astrocytes. Furthermore, using these cell culture apparatuses in the methods for cell cultivation, it may allow for increasing the areas of vascularized organoids through the porous cavities and it may be possible to create artificial blood-brain barriers (BBBs) or create human mimicking artificial BBBs.

Additionally, or alternatively, the cell culture membrane structure comprising the membrane made of polypropylene, polystyrene (PS), polydimethylsiloxane (PDMS), polycarbonate, polyethersulfone, polyethylene terephthalate, polyimide, or polytetrafluoroethylene, the average thickness of the membrane is in the range 8 - 12 µm, the membrane comprising the first surface being partially deformed by a plurality of membrane structure cavities protruding from the first surface, each of the plurality of membrane structure cavities has a cavity opening being each independently 50 - 3000 µm wide, each of the plurality of membrane structure cavities has an inner width being in the range of 50 - 3000 µm; and a plurality of through pores formed therein, the plurality of through pores of the membrane has an average pore size falling within a range from 0.2 µm to 10 µm, wherein the cell culture membrane structure further comprises at least one coating layer being made of a coating material, the at least one coating layer provided on the membrane such that the plurality of membrane structure cavities and the plurality of through pores of the membrane remain open, wherein the membrane material and the coating material are selected based on at least one type of cells to be cultured on the membrane and/or on the at least one coating layer, wherein the at least one coating layer comprises one first coating layer provided on a second surface of the membrane and one second coating layer provided on the first surface of the membrane, the second surface being on the opposite side of the membrane in respect to the first surface, the coating material of the one first coating layer and the one second coating layer is each independently selected from the group consisting of cell repellent compounds, such as pluronic F127, biolipidure, and Teflon; cell adherence promoter, or extracellular matrix; and the average thickness of each of the first coating layer and the second coating layer is in the range 1 - 50 µm, and the culture medium comprises organoids, spheroids, or human brain vascular endothelial cells and said another culture medium comprises human astrocytes. Alternatively, the culture medium comprises human astrocytes and said another culture medium comprises organoids, spheroids, or human brain vascular endothelial cells. It is to be understood that depending on which side of the membrane the membrane structure cavities are protruding from and hence, on which side of the membrane the cavity openings are (i.e., in the second chamber side of the membrane or on the opposite side of the membrane in respect of the second chamber), either the culture medium or the another culture medium comprises the organoids, spheroids, or human brain vascular endothelial cells, and, therefore, either the another culture medium or the culture medium comprises the human astrocytes. It is to be understood that the at least one type of cells (to be cultured on the membrane and/or on the at least one coating layer) comprises one first type of cells being the organoids, spheroids, or human brain vascular endothelial cells; and one second type of cells being the human astrocytes. These methods are beneficial, since the membrane made of polypropylene, polystyrene (PS), polydimethylsiloxane (PDMS), polycarbonate, polyethersulfone, polyethylene terephthalate, polyimide, or polytetrafluoroethylene is rigid, and organoids may be grown efficiently in the cavities. When the method comprises using a cell culture apparatus comprising a cell culture membrane structure comprising a coating material of a cell repellent compound, such as pluronic F127, biolipidure, and Teflon, organoids inside each cavity may be formed. A cell culture membrane structure comprising a coating material of cell adherence promoter, or extracellular matrix may form a cell monolayer on both sides of the membrane. The coatings of the porous membrane will help increase the physiological performance of barrier models. In addition, oxygen supply for the brain vascular endothelial cells and human astrocytes through the membrane and coating layers may be increased, being beneficial for the growth of the brain vascular endothelial cells and human astrocytes. Furthermore, using these cell culture apparatuses in the methods for cell cultivation, it may allow for increasing the areas of vascularized organoids through the porous cavities and it may be possible to create artificial blood-brain barriers (BBBs) or create human mimicking artificial BBBs.

Other features and advantages of the present disclosure will be apparent upon reading the following detailed description and reviewing the accompanying drawings. Other features and advantages of the present disclosure will be apparent upon reading the following detailed description and reviewing the accompanying drawings.

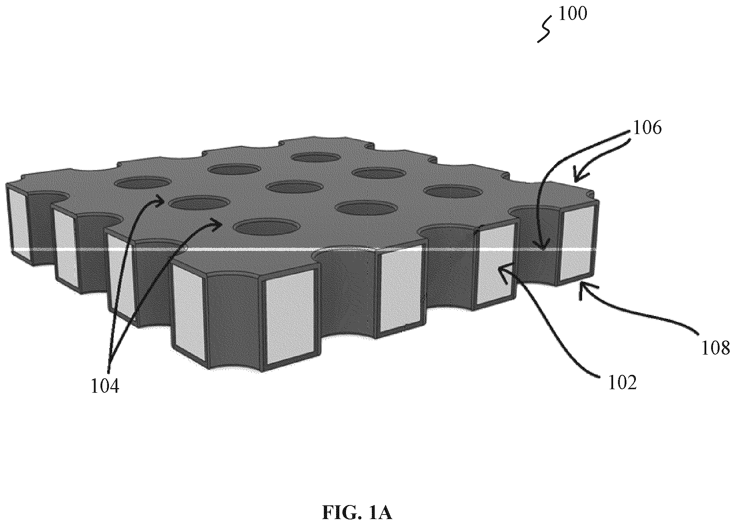

FIG. 1A shows a close up of a schematic perspective view of a cell culture membrane structure 100 in accordance with an exemplary embodiment (one or more membrane structure cavities protruding from the first surface are not shown). As shown in FIG. 1A, the structure 100 comprises a membrane 102 with through pores 104 formed therein, a first coating layer 106 provided on a second (top) side of the membrane 102, and a second coating layer 108 provided on a first (bottom) side of the membrane 102. The first and second coating layers 106 and 108 are provided on the second and first sides of the membrane 102 such that the through pores 104 remain open. The membrane 102 may be made of one of the following materials: polypropylene, polystyrene (PS), polydimethylsiloxane (PDMS), polycarbonate, polyethersulfone, polyethylene terephthalate, polyimide, polytetrafluoroethylene, silicone, silicon nitride. The first and second coating layers 106 and 108 may be made of the same coating material or different coating materials, depending on particular applications (in particular, depending on certain cells to be cultured on the first and second coating layers 106 and 108). The coating material for each of the first and second coating layers 106 and 108 may be selected from one of the following materials: cell adherence promoters such as endothelial cells, and epithelial cells; extracellular matrices such as a fibronectin, and collagen; cell repellent compounds such as pluronic F127, biolipidure, and Teflon; hydrogels such as matrigel, cultrex, Fibrinogen, and Alginate; silicone, polyacrylamide, PEG polymer, acrylamide, gelatin, gelatin derivative such as gelatin methacrylates, and hyaluronics; cellulose, nanocellulose, and fibrin. It is to be understood that extracellular matrices may be cell adherence promoters.

In embodiments, the membrane 102 may be free of the first or the second coating layer 106, 108 if it is required to study cell behavior only on the first or second side of the membrane 102. Thus, the presence of the two coating layers 106 and 108 should not be construed as any limitation of the present disclosure. Moreover, the first and second coating layers 106 and 108 may have the same or different thicknesses, depending on particular applications. In embodiments, the thicknesses of the first and second coating layers 106 and 108 may vary in the range 1- 50 µm, 8 - 75 µm, 8 - 50 µm, 8 - 40 µm, 8 - 30 µm, 8 - 20 µm, 8 - 12 µm, 15 - 110 µm, 20 - 100 µm, 30 - 100 µm, 40 - 100 µm, 50 - 100 µm, 60 - 100 µm, 70 - 100 µm, 80 - 100 µm, or 90 - 100 µm. In a preferred embodiment, the thicknesses of the first and second coating layers 106 and 108 vary in the range 1 - 50 µm. At the same time, care should be taken when selecting the thicknesses of the first and second coating layers 106 and 108, since an increase in the thickness of the whole structure 100 impacts the interaction of the cells to be cultured on the opposite first and second sides of the membrane 102.

As an addition or alternative, the first and second coating layers 106 and 108 may differ from each other in stiffness. For example, the first coating layer 106 may be made of low-stiffness silicone to provide a low-cell attachment surface for osteoblast cell culture, while the second coating layer 108 may be made of high-stiffness silicone for endothelial cell culture. Such a membrane structure 100 will allow one to implement a specified bone-marrow-on-chip model.

As for the through pores 104, their circular cross-section shape shown in FIG. 1A should not also be construed as any limitation of the present disclosure. In some embodiments, the cross-section shape of the through pores 104 may be triangular, rectangular, square, oval, or polygonal (e.g., hexagonal). The polygonal cross-section shape of the through pores 104 may refer to a convex or concave polygon. In some other embodiments, the through pores 104 may have different cross-section shapes, such as any combination of two or more of the above-mentioned cross-section shapes (e.g., the combination of circular and square cross-section shapes).

In embodiments, each of the through pores 104 may have a pore size varying within a predefined range of values, and a spacing (pitch) between the through pores 104 may vary within the same predefined range of values. For example, the pore size and spacing may both vary in the range 200 nm - 200 µm. In general, the predefined range of values for the pore size and the spacing between the through pores 104 is selected based on certain cells to be cultured on the first and second coating layers 106 and 108. It should be apparent that a pore density will depend on the pore size and spacing selected depending on particular applications. The pore density may, for example, be from 10³ to 10⁷ pores per millimeter square. Additionally, the pore sizes may be more or less than sizes of the cells to be cultured on the first and second coating layers 106 and 108. For example, if the pore size is less than the cell size, then it is possible to prevent the cells from migrating between the first and second sides of the membrane 102.

In embodiments in which cells of two different types are to be initially grown on the first coating layer 106 or the second coating layer 108 (i.e., on the same side of the membrane 102), each of the through pores 104 may have a pore size varying within a predefined range of values which is selected (e.g., based on sizes and/or elastic properties of the cells of the two types) such that the through pores 104 pass or filter the cells of one of the two types therethrough, while retaining (i.e. blocking) the cells of another of the two types. In this embodiment, such through pores 104 may be also spaced from each other by a distance varying within the same predefined range of values. By having such through pores 104 in the membrane 102, it is possible to sort out the cells according to their types.

In embodiments, the structure 100 may further comprise organic compounds and/or biomolecules bonded onto at least one of the first and second coating layers 106 and 108. The chemical agents may be represented by antifouling agents, such, for example, as pluronics or mono/polyethylene glycol (MEG/PEG) polymers, or lipids. The biomolecules may be represented by antigens, proteins, carbohydrates, antibody molecules, etc. By using such organic compounds and/or biomolecules, it is possible to provide an even larger spectrum of functionality of the structure 100.

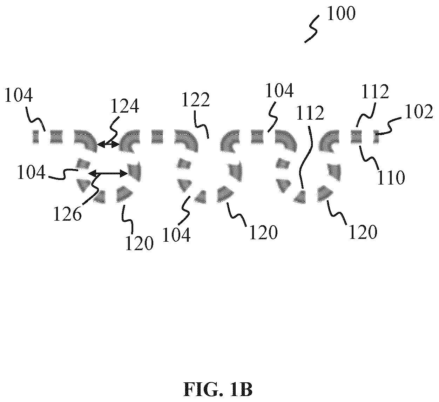

FIG. 1B shows a schematic cross-sectional side view of a cell culture membrane structure 100 in accordance with an exemplary embodiment. As shown in FIG. 1B, the structure 100 comprises a membrane 102 comprising a first surface 110 being partially deformed by one or more membrane structure cavities 120 protruding from the first surface; and one or more through pores 104 formed therein. A second surface 112 may be on the opposite side of the membrane in respect to the first surface. Each of the one or more membrane structure cavities 120 may have a cavity opening 122. The width 124 of a cavity opening 122 of each of the one or more membrane structure cavities 120 may be 50 - 3000 µm wide. Each of the one or more membrane structure cavities 120 may have an inner width 126 being in the range of 50 - 3000 µm. If the structure comprises at least one coating layer (not shown in the FIG. 1B), the at least coating layer is provided on the first 110 and/or second surface 112 (first and/or second sides) of the membrane 102 such that the one or more membrane structure cavities 120 and the through pores 104 remain open.

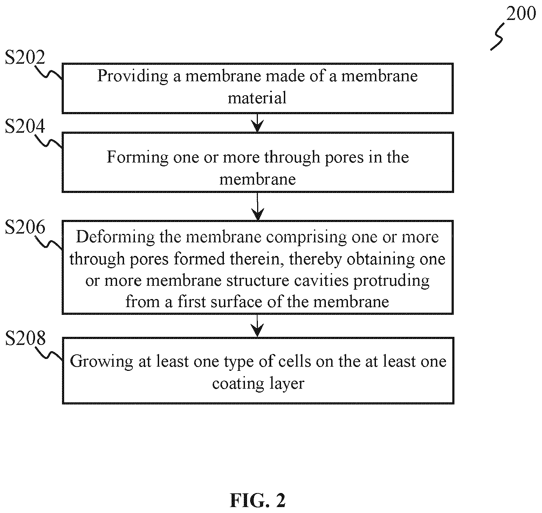

FIG. 2 shows a flowchart of a method 200 for producing the cell culture membrane structure 100. As shown in FIG. 2, the method 200 comprises step S202, which comprises providing the membrane 102 made of one of the above-mentioned membrane materials. In other words, the step S202 may comprises fabricating the membrane 102 of the selected membrane material. The membrane material is selected based on at least one type of cells to be cultured on the membrane. Method 200 further comprises step S204, in which one or more through pores 104 are formed in the membrane 102. Furthermore, the method 200 comprises step S206, in which deforming the membrane comprising one or more through pores formed therein is performed, thereby obtaining one or more membrane structure cavities 120 protruding from a first surface of the membrane. The method may further comprise a step S207 (not shown), which comprises applying at least one coating layer 106, 108 on the membrane such that the one or more membrane structure cavities and the one or more through pores of the membrane remain open, the at least one coating layer 106, 108 being made of a coating material. In these embodiments, the membrane material and the coating material are selected based on at least one type of cells to be cultured on the membrane and/or on the at least one coating layer. The at least one coating layer may comprise one first coating layer 106 provided on a second surface 112 of the membrane, the second surface 112 being on the opposite side of the membrane in respect to the first surface 110. The at least one coating layer may comprise one first coating 106 layer provided on a second surface 112 of the membrane 102, the second surface 112 being on the opposite side of the membrane 102 in respect to the first surface 110, and one second coating layer 108 provided on a first surface 110 of the membrane 102. The first and/or second coating layers 106 and 108 should be applied such that the through pores 104 and the one or more membrane structure cavities 120 of the membrane 102 remain open. As noted earlier, the membrane and coating materials are selected based on the cells to be cultured on the membrane and/or the at least one coating layers (first and/or second coating layers 106 and 108).

As also shown in FIG. 2, the method 200 may comprise a step S208, in which the cells of one or more types are grown on at least one coating layer (106 and 108). However, the step S208 is optional and may be omitted. In particular, the method 200 may end up with the step S206, thereby providing the cell culture membrane structure 100 manufactured as a roll or sheet with no cells grown thereon. The roll or sheet of the structure 100 may be provided to an end user who may then grow or culture the cells of desired type(s) (e.g., yeast cells, insect cells, mammalian cells, human cells, bacterial cells, tissue cells) on the structure 100 (e.g., on the first and second coating layers 106 and 108), for example, for the purpose of cell behavior study.

In embodiments, the step 204 of forming one or more through pores in the membrane is performed by using at least one of particle/fluid bombardment, etching and laser burning. These techniques may allow the through pores 104 to be formed more efficiently.

Additionally, or alternatively, the step 206 of deforming the membrane comprising one or more through pores formed therein may be performed by using vacuum thermal forming process. Additionally, or alternatively, the membrane material of the membrane used in the vacuum thermal forming process is selected from polypropylene, polystyrene (PS), polydimethylsiloxane (PDMS), polycarbonate, polyethersulfone, polyethylene terephthalate, polyimide, and polytetrafluoroethylene. One or more membrane structure cavities protruding from a first surface of the membrane may be obtained. It is to be understood that the one or more membrane structure cavities comprise one or more through pores in the membrane.

In embodiments, the step S206 may consist in applying the first coating layer 106 and/or the second coating layer 108 on the membrane 102 in a continuous manner. Then, the through pores 104 of the membrane 102 may be cleared by using etching and/or air or liquid pressurizing techniques. By using these techniques, it is possible to remove the coating material from the through pores 104 more efficiently.

In embodiments, the first coating layer 106 and/or the second coating layer 108 may be applied on the membrane 102 by using one of spin coating, blade coating, spray coating, immersion coating, brush coating, stamping, and electrospraying. It should be noted that clearing techniques, such as etching and/or air or liquid pressurizing, may not be required in case of using the spin coating process because spinning or centrifugal forces occurring in the spin coating process should clear the through pores 104 by their own.

In one embodiment, the method 200 may comprise a further step, in which electrically conductive materials or electrodes are printed or etched onto the membrane 102 before the step S207 of applying at least one coating layer 106, 108 on the membrane is performed. By using such electrodes, it is possible to study cell behavior in the presence of electric current.

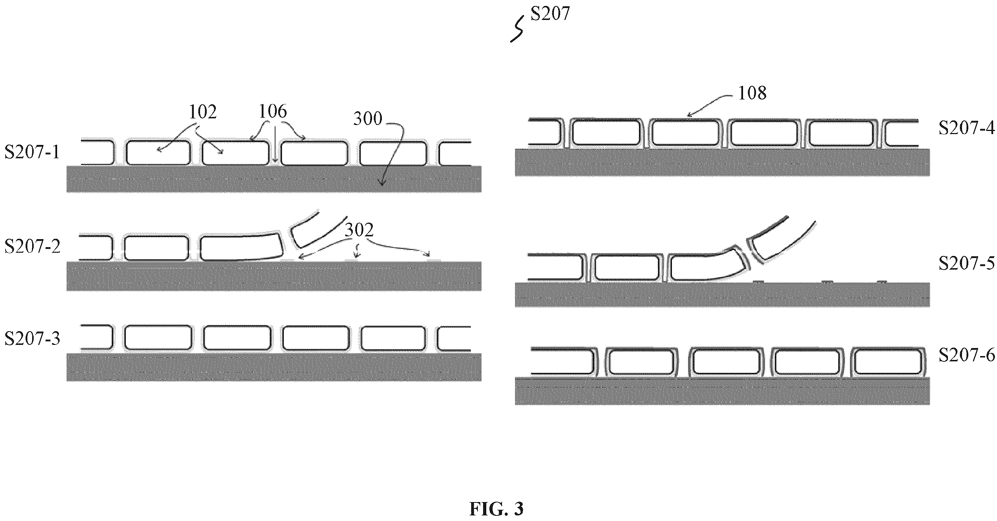

FIG. 3 explains an example on how to apply a dual coating (i.e., the first and second coating layers 106 and 108) on the membrane 102 by using the spin coating process in the step S207 of a method 200 (the one or more membrane structure cavities 120 of the membrane 102 are not shown). In this case, the step S207 comprises substeps S207-1 - S207-6. Let us assume that the first coating layer 106 is made of a first coating material (colored in light gray in FIG. 3), while the second coating layer 108 is made of a different second coating material (colored in black in FIG. 3). At first, in the substep S207-1, the membrane 102 is arranged on a working surface 300 (e.g., a flat wafer or substrate), and the first (top) side of the membrane 102 is spin-coated with the first coating material, thereby forming the first coating layer 106. Since the first coating layer 106 is provided as the thin meniscus of the first coating material on the membrane 102, a curing process may be also applied in the substep S207-1, such as heating or exposure to UV-light. Further, in the substep S207-2, the membrane 102 with the first coating layer 106 is peeled off the working surface 300. Due to the presence of the through pores 104 in the membrane 102, said peeling-off may lead to residual coating sticks 302 left on the working surface 300. The residual coating sticks 302 should be removed from the working surface 300. In the next substep S207-3, the membrane 102 is turned or flipped to access the second (bottom) side of the membrane 102 and arranged on the working surface 300. After that, the second side of the membrane 102 is spin-coated with the second coating material in the substep S207-4, thereby forming the second coating layer 108. Again, the second coating layer 108 may be also cured in the substep S207-4. The dual-coat membrane 102 or, in other words, the structure 100 is further peeled off the working surface 300, thereby leaving residual coating sticks of the second coating material (which may be removed if the working surface 300 is intended to be used further). In the last substep S207-6, the structure 100 is ready for further applications or processing.

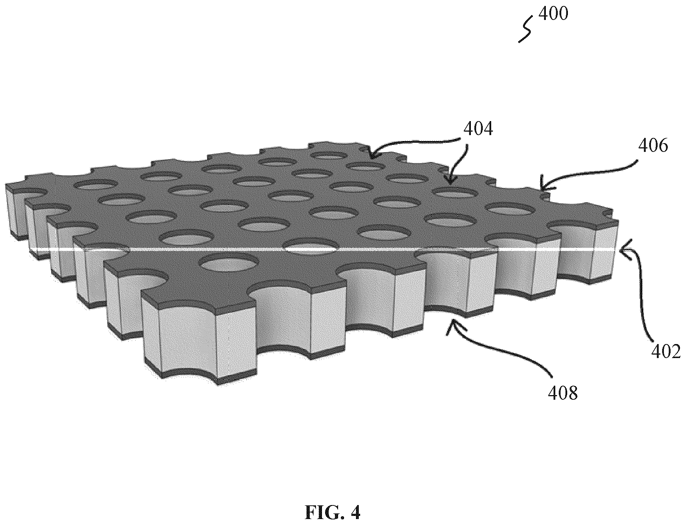

FIG. 4 shows a schematic perspective view of a cell culture membrane structure 400 in accordance with an exemplary embodiment (one or more membrane structure cavities protruding from the first surface are not shown). As shown in FIG. 4, the structure 400 comprises a membrane 402 with through pores 404 formed therein, a first coating layer 406 provided on a second (top) side of the membrane 402, and a second coating layer 408 provided on a first (bottom) side of the membrane 402. The first and second coating layers 406 and 408 are provided on the second and first sides of the membrane 402 such that the through pores 404 remain open. The membrane 402 and the coating layers 406 and 408 may be made of the same materials which are mentioned above with reference to the membrane 102 and the coating layers 106 and 108, respectively, which are included in the structure 100 shown in FIG. 1A. Similar to the coating layers 106 and 108, the coating layers 406 and 408 may be made of the same coating material or different coating materials and/or may have the same or different thicknesses, and/or one of the coating layers 406 and 408 may be not provided on the membrane 402, depending on particular applications (in particular, depending on certain cells to be cultured thereon). Moreover, the through pores 404 may be implemented in the same manner as the through pores 104 in the structure 100. As can be seen from FIG. 4, the structure 400 differs from the structure 100 in that it has no coating layer or material applied on the inner surface of each through pore 404, which is caused by the specifics of its production, as will be explained below.

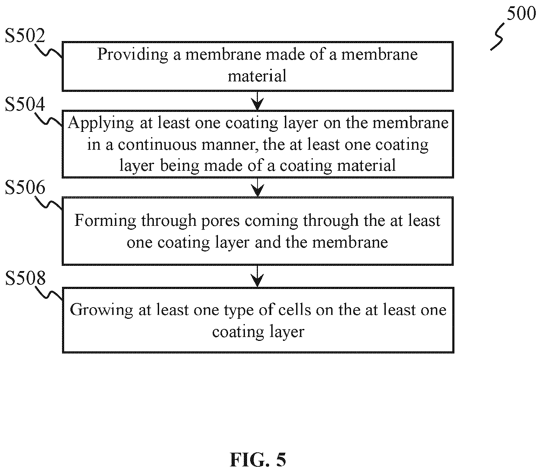

FIG. 5 shows a flowchart of a method 500 for producing the cell culture membrane structure 400. As shown in FIG. 5, the method 500 comprises step S502, in which the membrane 402 made of one of the above-mentioned membrane materials is provided. In other words, the step S502 may consist in fabricating the membrane 402 of the selected membrane material. Then, contrary to the method 200, the method 500 may proceed to an optional step S504, in which at least one coating layer (406, 408) is applied on the membrane 402 in a continuous manner. The step S504 may be performed by using one of spin coating, blade coating, spray coating, immersion coating, brush coating, stamping, and electrospraying. After that, the method 500 goes on to a step S506, in which the through pores 404 are formed in the membrane 402. If the optional step S504 is performed, and since the through pores 404 are formed after the first coating layer 406 and/or the second coating layer 408 are applied on the membrane 402, there is no coating layer on the inner surface of each through pore 404. As noted earlier, the membrane and coating materials are selected based on the cells to be cultured on the first and/or second coating layers 406 and 408.

Furthermore, the method 500 may comprise step S507 (not shown), preferably comprises step S507, in which the membrane is deformed, thereby obtaining one or more membrane structure cavities 120 protruding from a first surface 110 of the membrane. Step S507 may be performed before the optional step S504, before step S506, or after step S506. The deforming the membrane is performed such that one or more membrane structure cavities protruding from a first surface of the membrane is obtained, wherein at least a part, preferably each, of the one or more membrane structure cavities 120 has a cavity opening 122. In addition, if S507 is performed after S506, S507 is performed such that at least a part of, preferably each of, the one or more through pores 402 remain open. The cell culture membrane structure may be adapted for certain one or more types of cells to be cultured thereon, in addition in the one or more membrane structure cavities.

Additionally, or alternatively, the membrane provided in step S502 is made of a membrane material, the membrane comprising a first surface being partially deformed by one or more membrane structure cavities protruding from the first surface.

As also shown in FIG. 5, the method 500 comprises a step S508, in which the cells of one or more types are grown on at least one of the first and second layer 406 and 408. However, similar to the step S208 of the method 200, the step S508 of the method 500 is optional and may be omitted for the same reasons. In other words, the method 500 may end up with the step S506, thereby providing the cell culture membrane structure 400 manufactured as a roll or sheet with no cells grown thereon. The roll or sheet of the structure 400 may be provided to an end user who may then grow or culture the cells of desired type(s) (e.g., mammalian cells, human cells, bacterial cells, tissue cells, etc.) on the structure 400 (e.g., on the first and second coating layers 406 and 408), for example, for the purpose of cell behavior study.

It should be also noted that the step S506 of the method 500 may be implemented similar to the step S204 of the method 200. That is, the through pores 404 may be formed by using at least one of particle/fluid bombardment, etching and laser burning.

In one embodiment, similar to the method 200, the method 500 may comprise a further step, in which electrically conductive materials or electrodes are printed or etched onto the membrane 402 before the step S504. By using such electrodes, it is possible to study cell behavior in the presence of electric current.

Let us now give a non-limitative practical example of how to apply a dual coating on a desired membrane, depending on particular applications. It should be noted that the dual coating discussed in the practical example may be applied either in the optional step S207 of the method 200 or in the optional step S504 of the method 500, meaning that the desired membrane may correspond to the membrane 102 or 402, respectively. In other words, the dual coating discussed in the practical example may be applied after or before through holes are formed in the desired membrane.

### Practical example 1

This practical example relates to a vascularized astrocyte spheroid-on-chip model, in which a spheroid formation compartment (one or more membrane structure cavities protruding from the first surface of the membrane) of a chip (cell culture apparatus) is assumed to require that the desired membrane structure cavities has one (e.g., at least the inner surface of cavities on the top of the membrane, i.e. the second surface or the apical surface of the membrane) low-adhesion surface to allow for spheroid formation and another (e.g., bottom, i.e. the first surface of the membrane or the basal surface of the membrane) high-adhesion surface to allow for endothelial cell attachment.

Given this, the membrane's cavities formation (e.g., step S206 or S507) and the dual-coating process itself (e.g., step S207 or S504) may be performed as follows:
1. A membrane comprising through pores formed therein and having a thickness of 8 microns is clamped between a negative mold in a vacuum thermal forming machine for 5 minutes at 120 degrees Celsius to form the membrane structure cavities on the membrane (a first surface of the membrane being partially deformed by one or more membrane structure cavities protruding from the first surface).
2. The formed membrane (comprising the cavities) is then transferred to the chip (cell culture apparatus) lacking the membrane by integrating it into an injection molding process for preparing the chip (cell culture apparatus) thus attaching the membrane on top of the second chamber of a cell culture apparatus, or by bonding it to an insert (cell culture membrane structure comprising an insert wall such as a bottomless hollow tube) lacking the membrane and then attaching the thus formed insert (cell culture membrane structure comprising an insert wall and the membrane) on top of the second chamber of a cell culture apparatus.
3. The first coating layer is applied using a 100 µg/mL fibronectin solution in PBS by loading the 100 µg/mL fibronectin solution in PBS into a microchannel of the cell culture apparatus (i.e., via one of the at least two culture medium reservoirs and towards and in the second chamber) to coat the bottom surface (i.e., the first surface of the membrane or the basal surface of the membrane) of the formed membrane. This yields a cell promoter layer (coating layer) for the first cell line (i.e., endothelial cells) attachment.
4. The second coating layer is applied using a 4% solution of pluronic in water, which is spin-coated on the top surface of the membrane (i.e., the second surface or the apical surface of the membrane, including the inner surface of structure cavities), with the spin-coating parameters being as follows: spin speed: 1000 rpm; spin-coating time: 90 seconds. This yields a low-adhesion surface (coating layer) in the membrane structure cavities (i.e., the second surface).
5. Spheroids are formed by loading an astrocyte cell suspension of 2×10E6 cells/mL to the top surface of the membrane (i.e., feeding culture medium via the first chamber or the cell culture membrane structure comprising an insert wall (i.e., an insert) towards the membrane) that has cavity openings (of the formed cavities). The loaded chip is then centrifuged as follows: spin speed: 2000 rpm; centrifugation time: 90 seconds. This will settle cells into the membrane structure cavities having a low-adhesion surface (coating layer) and form spheroids.
6. After spheroid formation, brain microvascular endothelial cells (HPBMEC, 2×10E6 cells/mL solution) are loaded into a microchannel of the cell culture apparatus (i.e., via one of the at least two culture medium reservoirs and towards and in the second chamber) and the HPBMEC adhere to the bottom surface of the membrane (i.e., the first surface of the membrane or the basal surface of the membrane having the coating layer of fibronectin that was applied in step 3), thus creating a coculture model of blood-brain-barrier.

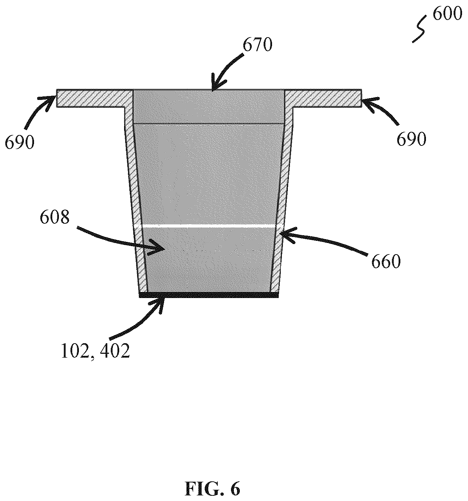

FIG. 6 shows a schematic cross-sectional view of a cell culture membrane structure 600 in accordance with an exemplary embodiment (one or more membrane structure cavities protruding from the first surface are not shown). As shown in FIG. 6A, contrary to the cell culture membrane structure 100 and 400, the cell culture membrane structure 600, which may be any cell culture membrane structure 600 as defined in the present disclosure, further comprises an insert wall 660 attached to the cell culture membrane structure, an inner insert cavity 608, and an insert opening 670; wherein the inner insert cavity 608 is surrounded by the insert opening 670, the membrane 102, 404, and the insert wall 660; the insert wall 660 extending from the membrane to the insert opening 670; and the insert cavity 608 is located between the insert opening 670 and the membrane 102. The membrane 102, 402 comprises a first surface 110 being partially deformed by one or more membrane structure cavities 120 (not shown) protruding from the first surface; and one or more through pores 104 formed therein (not shown). The cell culture membrane structure 600 may comprise one or more protrusions 690 protruding from the upper part of the insert wall 660. In embodiments, the first surface of the membrane is located on the opposite side of the membrane in respect to the inner insert cavity 608. In embodiments, the first surface of the membrane is located on the same side of the membrane in respect to the inner insert cavity 608. The inner insert cavity 608 may be used, for example, to place different biological objects (e.g., an organoid or spheroid) on the top side of the membrane 102, 402 of the cell culture membrane structure 600 for research purposes or for growing biological objects (e.g., a cell, an organoid, or a spheroid). In embodiments, the insert wall 660 is a bottomless hollow tube.

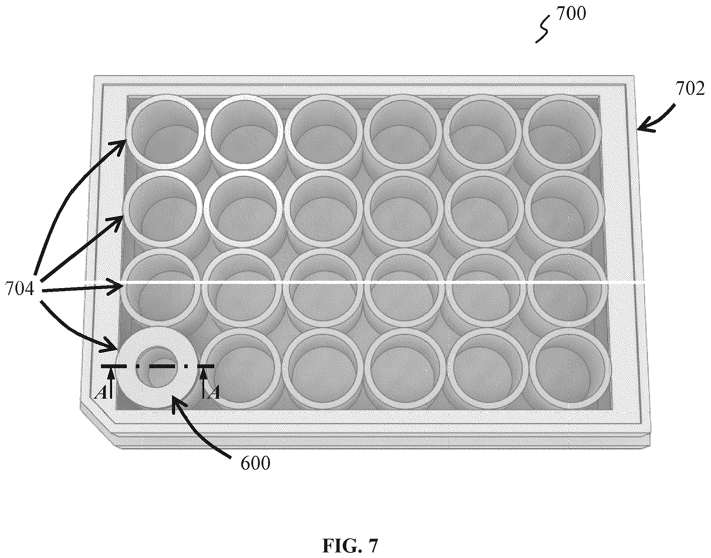

FIG. 7 shows a schematic perspective view of a cell culture plate 700 in accordance with an exemplary embodiment. As shown in FIG. 7, the cell culture plate 700 comprises a housing 702, which may be ridged, encompassing one or more wells 704. Each well 704 may be shaped as a cylinder and arranged to retain a cell culture media and one or more cell culture membrane structures 600 each being a replaceable insert configured to be immersed into the cell culture media. Each of the inserts 600 may comprise the cell culture membrane structure 100 or the cell culture membrane structure 400. The cell culture plate 600 makes it possible to grow cells of desired type(s) on a large amount of cell culture membrane structures, like the structures 100 and/or 400 and/or 600, simultaneously and under the same growing conditions. It should be apparent to those skilled in the art that the number, shape and spacing of the wells 704 shown in FIG. 7 are for illustrative purposes only and should not be construed as any limitation of the present disclosure.

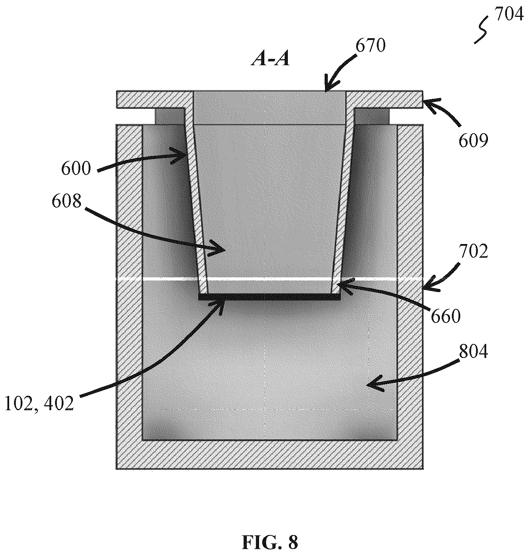

FIG. 8 shows a schematic cross-sectional view of one well 704 of the cell culture plate 700, as taken along the line A-A of FIG. 7. As shown in FIG. 8, the well 704 comprises a body 802 with an inner cavity 804 which may be filled with the cell culture media. The cell culture membrane structure being an insert 600 is immersed into the inner cavity 804 and comprises a membrane 102, 402 attached to the bottom of the insert 600 (the one or more membrane structure cavities 120 protruding from the first surface of the membrane, and the one or more through pores 104, 404 formed in the membrane are not shown). The insert 600 comprises an insert wall 660 attached to the cell culture membrane structure (being the insert), an inner insert cavity 608, and an insert opening 670; wherein the inner insert cavity 608 is surrounded by the insert opening 670, the membrane 102, and the insert wall 660; the insert wall 660 extending from the membrane to the insert opening 670; and the insert cavity 608 is located between the insert opening 670 and the membrane 102. The cell culture membrane structure 600 may comprise one or more protrusions 690 protruding from the upper part of the insert wall 660. In embodiments, the insert wall 660 is a bottomless hollow tube. The cell culture membrane structure 600 (being an insert) may be any cell culture membrane structure 100, 400 as defined in the present disclosure, depending on particular applications. The inner insert cavity 608 may be used, for example, to place different biological objects (e.g., an organoid or spheroid) on the top side of the membrane 102, 402 of the cell culture membrane structure 600 for research purposes or for growing biological objects (e.g., a cell, an organoid, or a spheroid).

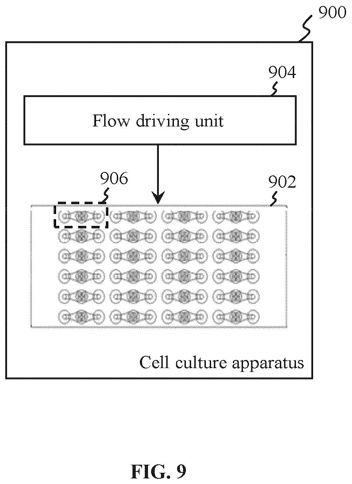

FIG. 9 shows a block diagram of a cell culture apparatus 900 in accordance with an exemplary embodiment. The apparatus 900 is intended for the above-mentioned microfluidic cell culture. As shown in FIG. 9, the apparatus 900 comprises a cell culture plate 902. The cell culture plate 902 comprises one or more cell culture modules 906 and may be configured to fit the standard 96, 384, or 1536 microtiter plates (American National Standards Institute (ANSI), having standardized footprint dimensions, plate height, flange dimensions, chamfers, side wall rigidity, and well positions). The apparatus 900 may further comprise a flow driving unit 904 configured to cause the culture medium to flow, in each cell culture module of the one or more cell culture modules, between the at least two culture medium reservoirs 10202, 10204 via the at least two flow channels 10210, 10212 and the second chamber 10208, as will be described below in more detail. The flow driving unit 904 is configured to cause a culture medium to flow in each cell culture module 906, as will be described below in more detail. It should be noted that the number, arrangement, and interconnection of the constructive elements constituting the apparatus 900, which are shown in FIG. 1, are not intended to be any limitation of the present invention, but merely used to provide a general idea of how the constructive elements may be implemented within the apparatus 900. For example, the cell culture plate 902 may be replaced with two or more cell culture plates, and the flow driving unit 904 may be replaced with two or more flow driving units each configured to control a culture medium flow in one of the cell culture plates.

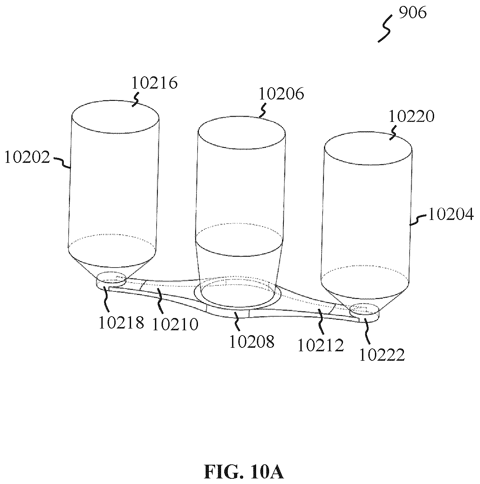

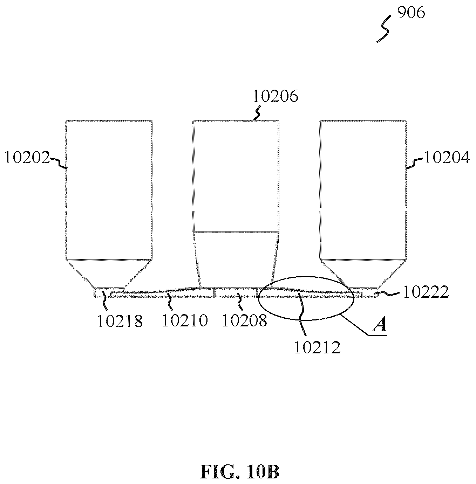

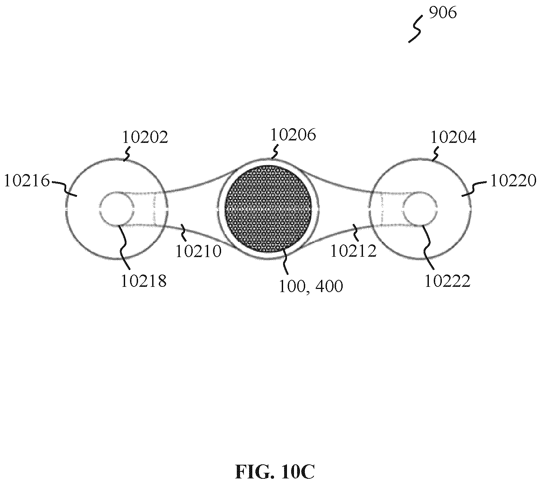

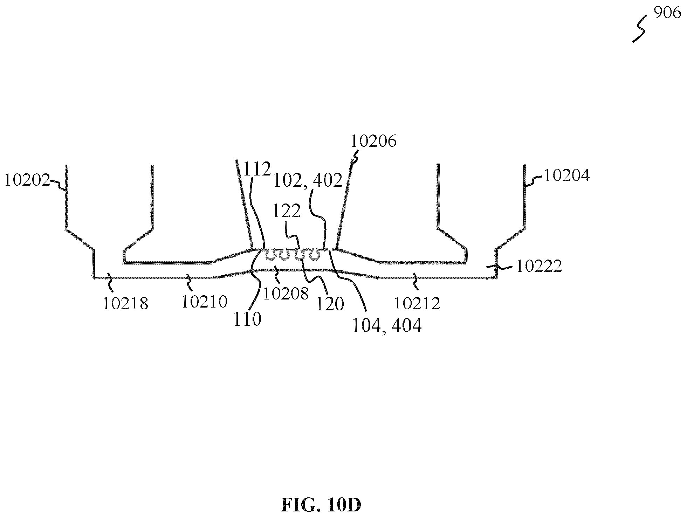

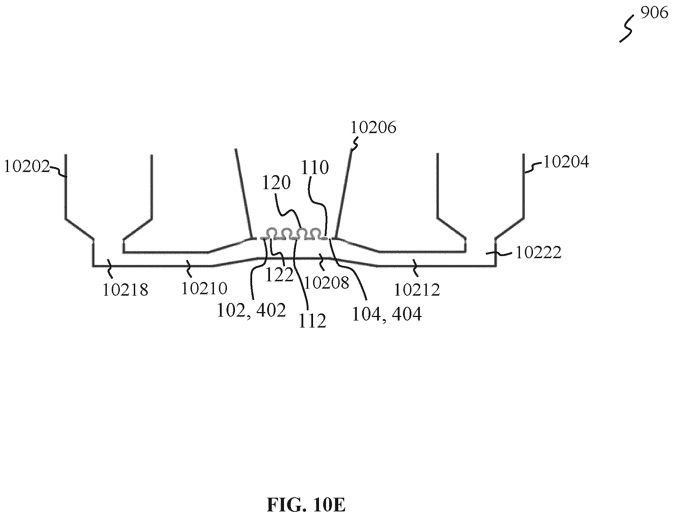

FIGs. 10A-10E show different schematic views of the cell culture module 906 included in the apparatus 900 in accordance with exemplary embodiments. More specifically, FIG. 10A shows a schematic perspective view of the cell culture module 906, FIG. 10B shows a schematic side view of the cell culture module 906, FIG. 10C shows a schematic top view of the cell culture module 906, FIG. 10D shows a schematic side view of a cell culture module 906, and FIG. 10E shows a schematic side view of a cell culture module 906. As shown in FIGs. 10A-10C, the cell culture module 906 comprises a first culture medium reservoir 10202; a second culture medium reservoir 10204; a second (basal or bottom) chamber 10208; a first flow channel 10210; and a second flow channel 10212. The cell culture module 906 may further comprise a first (apical or top) chamber 10206 configured to be arranged between the at least two culture medium reservoirs 10202, 10204 such that the first chamber 10206 and the at least two culture medium reservoirs 10202, 10204 are aligned with each other in a first direction, and a cell culture membrane structure 100, 400 (not shown in Figs. 10A-10B) configured to be arranged between the first chamber 10206 and the second chamber 10208; or a cell culture membrane structure 600 (not shown in FIGs.10A-10C). The cell culture membrane structures 100, 400, and 600 comprise a first surface being partially deformed by one or more membrane structure cavities 120 (not shown) protruding from the first surface, and one or more through pores 104, 404 formed therein (not shown in FIGs.10A-10C). It should be again noted that the present disclosure is not limited to the shown number, arrangement, and interconnection of the constructive elements of the cell culture module 906. In some embodiments, there may be more than two medium culture reservoirs, thereby leading to more than two flow channels. In other embodiments, there may be more than two apical chambers and more than two basal chambers between the two culture reservoirs, thereby also increasing the number of the flow channels connecting the two culture reservoirs through the apical chambers and the basal chambers. The first culture medium reservoir 10202 has a top part with an inlet 10216 for the culture medium and a bottom part with an outlet 10218 for the culture medium. Similarly, the second culture medium reservoir 10204 has a top part with an inlet 10220 for the culture medium and a bottom part with an outlet 10222 for the culture medium. The first and second culture medium reservoirs 10202 and 10204 may be implemented as identical hollow tubes. Although FIGs. 10A and 10C show that each of the first and second culture medium reservoirs 10202 and 10204 has a circular cross-section, this should not be construed as any limitation of the present disclosure; in some embodiments, any other cross-sectional shapes, such as polygonal, oval, etc., are possible, if required and depending on particular applications. Moreover, the bottom part of each of the first and second culture medium reservoirs 10202 and 10204 may have a different profile, for example, a positively tapered profile as shown in FIGs. 10A and 10B.

In embodiments of the apparatus, the first chamber 10206 is configured to be arranged between the at least two culture medium reservoirs 10202, 10204 such that the first chamber 10206 and the at least two culture medium reservoirs 10202, 10204 (the first and second culture medium reservoirs 10202, 10204) are aligned with each other in a first (horizontal) direction. The cell culture membrane structure 100, 400 (not shown in Figs. 10A-10B) being configured to be arranged between the at least two culture medium reservoirs (the first chamber 10206 and the second chamber 10208) such that the first chamber 10206 and the second chamber 10208 are in flow communication with each other via the through pores 104, 404 (not shown). The first chamber 10206 may be implemented as a bottomless hollow tube, for example, with a positively tapered profile (as shown in FIGs. 10A and 10B) or with a uniform cross-section. Again, the shown circular cross-section of the first chamber 10206 is for illustrative purposes only and should not be considered as any limitation of the present disclosure. This arrangement of the first chamber 10206 and the cell culture membrane structure 100, 400 enables the first chamber 10206 and the cell culture membrane structure 100, 400 to be arranged at the apparatus when in use, and the first chamber 10206 and the cell culture membrane structure 100, 400 may be removed when the apparatus is not in use, provided that when in use the first chamber 10206 and the second chamber 10208 are in flow communication with each other via the through pores 104, 404 (not shown). The first chamber 10206 and the cell culture membrane structure 100, 400 may be attached, e.g., using a snap-fit, to the apparatus when the apparatus is in use, and the first chamber 10206 and the cell culture membrane structure 100, 400 may be detached, e.g., using the snap-fit, from the apparatus when the cell culture apparatus is not in use.

In embodiments of the apparatus (not shown in FIGs.10A-10C), the cell culture membrane structure 600 is configured to be arranged between the at least two culture medium reservoirs 10202, 10204 (the first and second culture medium reservoirs 10202, 10204) such that the cell culture membrane structure 600 and the at least two culture medium reservoirs 10202, 10204 are aligned with each other in a first direction, the membrane 102, 402 of the cell culture membrane structure 600 being arranged between the inner insert cavity 708 and the second chamber 10208 such that the inner insert cavity 708 and the second chamber 10208 are in flow communication with each other via the through pores 104, 404. This arrangement of cell culture membrane structure 600 enables the cell culture membrane structure 600 to be arranged at the apparatus when in use, and the cell culture membrane structure 600 may be removed when the apparatus is not in use, provided that when in use the inner insert cavity 708 and the second chamber 10208 are in flow communication with each other via the through pores 104, 404 (not shown) of the cell culture membrane structure 600. The cell culture membrane structure 600 may be attached, e.g., using a snap-fit, to the apparatus when the apparatus is in use, and the cell culture membrane structure 600 may be detached, e.g., using the snap-fit, from the apparatus when the cell culture apparatus is not in use.

The second chamber 10208 may be arranged under the first chamber 10206 and aligned with the first chamber 206 in a second (vertical) direction. The second chamber 10208 may have a cross-section corresponding to the cross-section of the first chamber 10206. The second chamber 10208 has a bottom part having two or more lateral holes (not shown in FIGs. 10A-10C). As shown in FIGs. 10A-10C, the second chamber 10208 has dimensions significantly smaller than the first chamber 10206 and the cell culture membrane structure 600 (not shown). For example, if the first chamber 10206 or the cell culture membrane structure 600 has a height of about 7 mm, then the height of the second chamber 10208 may fall within a range from 50 µm to 150 µm (at height values higher than 150 µm, the second chamber 10208 may lose microfluidic properties). Such height values of the first chamber 10206, the cell culture membrane structure 600, and the second chamber 10208 (in addition to the heights of the reservoirs 10202 and 10204) make the cell culture module 906 more compact, thereby reducing the total dimensions of the apparatus 900.

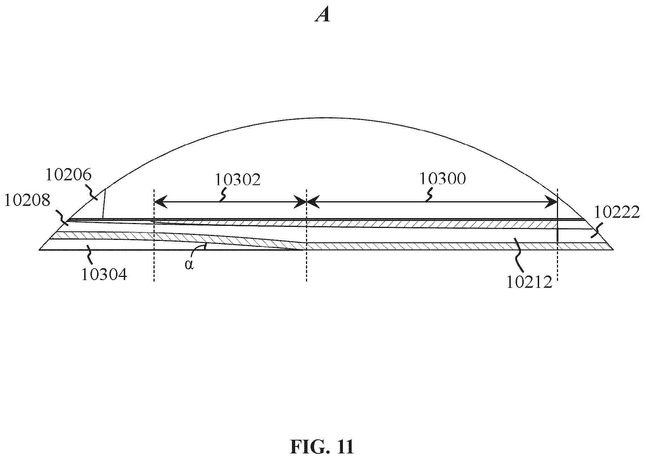

The membrane 102, 402 of the cell culture membrane structure 100, 400 (see FIG. 10C) comprises through pores and a first surface 110 (not shown) being partially deformed by one or more membrane structure cavities 120 (not shown) protruding from the first surface; and the cell culture membrane structure 100, 400 is arranged (when in use) between the first chamber 10206 and the second chamber 10208 such that the first chamber 10206 and the second chamber 10208 are in flow communication with each other via the through pores. Alternatively, the membrane 102, 402 of the cell culture membrane structure 600 comprises through pores (this alternative is not shown in FIG 11C) and the cell culture membrane structure 600 is arranged (when in use) between the inner insert cavity 708 and a second chamber 208 such that the inner insert cavity 708 and the second chamber 208 are in flow communication with each other via the through pores (not shown in FIG. 10C). The membrane 102, 402 may be made of a membrane material such as artificial polymers (e.g., polyester (PET), polypropylene, polystyrene (PS), polydimethylsiloxane (PDMS), polycarbonate, polyethersulfone, polyethylene terephthalate, polyimide, polytetrafluoroethylene), silicone, silicon nitride, plastic, protein, natural polymers, metallic polymers or carbohydrates (e.g., cellulose), and may be formed by using one of the following methods: lithography, stamping, casting, electrospinning or *in situ* polymerization. The through pores may have different cross-sectional shapes, such, for example, as triangular, rectangular, square, oval, or polygonal (e.g., hexagonal). The polygonal cross-section shape of the through pores may refer to a convex or concave polygon. In some embodiments, the through pores may have different cross-sectional shapes, such as any combination of two or more of the above-mentioned cross-sectional shapes (e.g., the combination of circular and square cross-section shapes). Furthermore, each of the through pores may have a pore size varying within a predefined range of values. For example, the pore size may vary from 0.2 µm to 10 µm. In general, the predefined range of values for the pore size (as well as the spacing between the through pores) is selected based on certain cells to be deposited on the membrane 102, 402. More specially, the pore sizes may be more or less than sizes of the cells to be deposited on the membrane. For example, if the pore size is less than the cell size, then it is possible to prevent the cells from migrating between the basal and apical surfaces of the membrane 102, 402.

The first and second flow channels 10210 and 10212 may be microchannels providing the passage of the culture medium between the first and second culture medium reservoirs 10202 and 10204 through the second chamber 10208. In particular, the first flow channel 10210 connects the outlet 10218 of the bottom part of the first culture medium reservoir 10202 to one or more of the lateral holes arranged on the left side of the bottom part of the second chamber 10208. The second flow channel 10212 connects the outlet 10222 of the bottom part of the second culture medium reservoir 10204 to one or more of the lateral holes arranged on the right side of the bottom part of the second chamber 10208. Each of the first and second flow channels 10210 and 10212 may have a longitudinal section and a cross-section which allow one to achieve required flow characteristics (e.g., an appropriate flow rate). For example, each of the first and second flow channels 10210 and 10212 may a variable channel height and/or a variable channel width which increase (e.g., gradually) towards the second chamber 10208.

In embodiments, the apparatus comprises any cell culture membrane structure 600 as disclosed in the present disclosure, and the apparatus further comprises a first chamber 10206 configured to be arranged between the at least two culture medium reservoirs 10202, 10204 such that the first chamber 10260 and the at least two culture medium reservoirs 10202, 10204 are aligned with each other in the first direction, wherein the cell culture membrane structure 600 being configured to be detachably inserted in the first chamber 10206, the second chamber 10208 being arranged under and aligned with the cell culture membrane structure 600 in the second direction. These embodiments may enable a cell culture membrane structure 600 to be inserted in a first chamber 10260 when the apparatus is in use (such that the inner insert cavity 708 and the second chamber 208 are in flow communication with each other via the through pores), and may be removed from the first chamber 10260 when the apparatus is not in use.

In embodiments, the apparatus further comprises a flow driving unit 904 configured to cause the culture medium to flow, in each cell culture module of the one or more cell culture modules, between the at least two culture medium reservoirs 10202, 10204 via the at least two flow channels 10210, 10212 and the second chamber 10208. It is to be understood that the flow driving unit 904 may cause the culture medium to flow also to the first chamber 10206 or the inner insert cavity 708 of the cell culture membrane structure 600 via the one or more through pores 104, 404 of the membrane 102, 402.

In embodiments, the apparatus comprises a plurality of cell culture modules 906 arranged adjacent to each other.

Both FIGs. 10D-10E shows a schematic side view of the cell culture module 906 in accordance with exemplary embodiments. As for FIG. 10B, the cell culture modules 906 in FIGs. 10D-10E comprises the first culture medium reservoir 10202 and the second culture medium reservoir 10204, each having a top part and a bottom part, the top part having an inlet 10216, 10220, for a culture medium and the bottom part having an outlet 10218, 10222 for the culture medium. Furthermore, the cell culture module 906 comprises a first chamber 10206 configured to be arranged between the at least two culture medium reservoirs such that the first chamber and the at least two culture medium reservoirs are aligned with each other in a first direction, and a cell culture membrane structure 100, 400 (not shown) configured to be arranged between the first chamber and a second chamber 10208 such that the first chamber and the second chamber are in flow communication with each other via the through pores 104, 404 (not shown), wherein the second chamber 10208 being arranged under and aligned with the first chamber 10206 in a second direction, the second direction having an angle being more than 0 degrees but 90 degrees or less in respect to the first direction (preferably the second direction being perpendicular to the first direction), the second chamber having a bottom part having at least two lateral holes (not shown); and at least two flow channels 10210, 10212, each of the at least two flow channels being configured to pass a fluid or cell culture media therethrough, and each of the at least two flow channels 10210, 10212 connecting the outlet 10218, 10222 of the bottom part of one of the at least two culture medium reservoirs 10202, 10204 to one of the at least two lateral holes of the bottom part of the second chamber 10208. Furthermore, FIGs. 10D-10E show a first surface 110 of the membrane 102, 402 being partially deformed by one or more membrane structure cavities 120 protruding from the first surface, and cavity opening 122 of each of the one or more membrane structure cavities 120. The second surface 112, and one or more through pores 104, 404 formed in the membrane are also shown in FIGs.10D-E. In FIG. 10D is shown an exemplary embodiment, wherein the cavity opening 122 of each of the one or more membrane structure cavities 120 of the cell culture membrane structure is located on the apical surface of the cell culture membrane structure (i.e., the apical surface is the second surface 112 of the membrane and the basal surface is the first surface 110 of the membrane). In contrast to FIG. 10D, FIG. 10E shows an exemplary embodiment, wherein the cavity opening 122 of each of the one or more membrane structure cavities 120 of the cell culture membrane structure is located on the basal surface of the cell culture membrane structure (i.e., the basal surface is the second surface 112 of the membrane and the apical surface is the first surface 110 of the membrane). Cells, organoids, and/or spheroids may be formed in the one or more membrane structure cavities 120. It should be noted that for example the one or more membrane structure cavities 120 are not necessary in scale. It should be apparent to those skilled in the art that the number, shape and spacing of the one or more membrane structure cavities 120, cavity opening(s) 122, and through pores 104, 404 shown in FIGs. 10D-10E are for illustrative purposes only and should not be construed as any limitation of the present disclosure.

FIG. 11 shows a sectional view of the second flow channel 10212 of the cell culture module 906, as taken within the area delimited by oval A in FIG. 10B in accordance with an exemplary embodiment. It should be noted that the first flow channel 10210 has a similar sectional view (one just needs to mirror the shown sectional view). As shown in FIG. 11 the second flow channel 10212 comprises a first channel portion 10300 and a second channel portion 10302. The first channel portion 10300 extends from the outlet 10222 of the bottom part of the second culture medium reservoir 10204 and is parallel to the first (horizontal) direction. The second channel portion 10302 connects the first channel portion 10300 to the lateral hole(s) (not shown in FIG. 11) arranged on the right side of the second chamber 10208. The second channel portion is tilted up (i.e., at a tilting angle α to the first direction) such that the bottom part of the second chamber 10208 may be arranged higher than the bottom part of the second culture medium reservoir 10204 (i.e., higher than the end of the outlet 10222). This tilt of the second channel portion 10302 is provided by a small "hill" 10304 which supports the second chamber 10208. The tilting angle α of the second channel portion 10302 relative to the first (horizontal) direction preferably falls within a range of 5 degrees to 45 degrees to provide appropriate flow characteristics. In other embodiments, each of the first and second flow channels 10210 and 10212 may be fully tilted up at the tilting angle α, starting from the outlet of the corresponding culture medium reservoir (i.e., there may be no first channel portion parallel to the first (horizontal) direction).

In embodiments, each of the second chamber 10208, the first flow channel 10210, and the second flow channel 10212 may be coated, from inside, with a cell adhesion enhancing layer. The cell adhesion enhancing layer may be made of a hydrophilic material. Some non-restrictive examples of the hydrophilic material may include Matrigel, fibronectin, hyaluronic acid, different types of collagen (e.g., a type I collagen which is suitable for creating a blood-brain barrier cell culture model based on the apparatus 900), laminins, tenascin, elastane, nanocellulose with a host of different molecules. Such cell adhesion enhancing layers allow for less cells to be used in an experiment and more precise deposition of the cells on the basal surface of the membrane 102, 402.

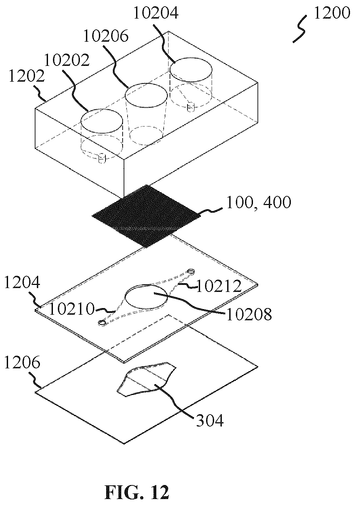

FIG. 12 shows a schematic exploded view of a layered structure 1200 that represents one possible implementation example of the cell culture module 906. As shown in FIG. 12, the layered structure 1200 comprises a first layer 1202, a second layer 1204, and a third layer 1206. The first layer 1202 comprises the first and second culture medium reservoirs 10202 and 10204, as well as the first chamber 10206. The second layer 1204 comprises the second chamber 10208 arranged exactly under the cell culture membrane structure 100, 400, as well as the flow channels 10210 and 10212. The cell culture membrane structure 100, 400 is arranged between the first chamber 10206 and the second chamber 10208 to provide the fluid communication therebetween via the through pores. The cell culture membrane structure 100, 400 comprises a membrane 102, 402 (not shown) comprising a first surface 110 being partially deformed by one or more membrane structure cavities 120 (not shown) protruding from the first surface; and one or more through pores 104 formed therein (not shown). Moreover, the inlets of the flow channels 10210 and 10212 are arranged exactly under the outlets 10218 and 10222 (not shown in FIG. 12) of the culture medium reservoirs 10202 and 10204, respectively, to provide the fluid communication between the reservoirs 10202 and 10204 and the flow channels 10210 and 10212, respectively. The third layer 1206 comprises the small "hill" 10304 which is formed such that the appropriate tilting angle α of each of the flow channels 10210 and 10212 is provided. It should be noted that each of the first layer 1202, the second layer 1204, and the third layer 1206 may be made of roomtemperature-vulcanizing (RTV) silicone (e.g., RTV615) or polydimethylsiloxane (PDMS). Moreover, the layers 1202-1206 may be attached to each other in the order shown in FIG. 12 by using adhesive or plasma bonding. The cell culture modules 906 thus configured may be attached to each other by the using the same adhesive or plasma bonding to implement the cell culture plate 902 of the apparatus 900.

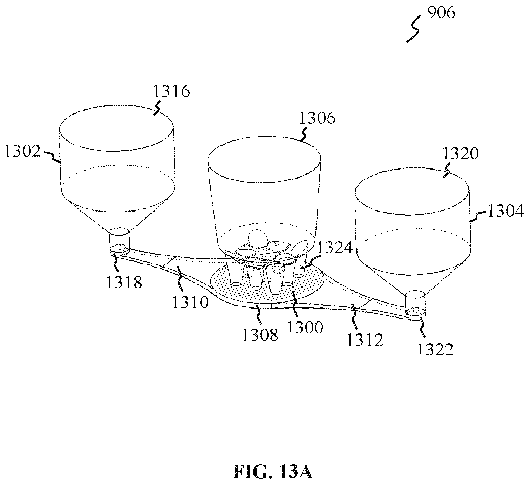

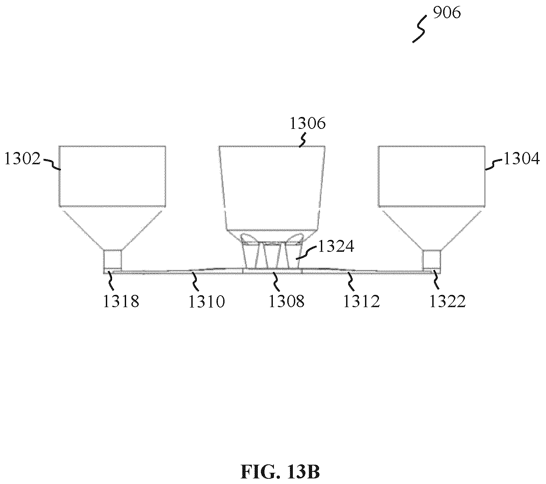

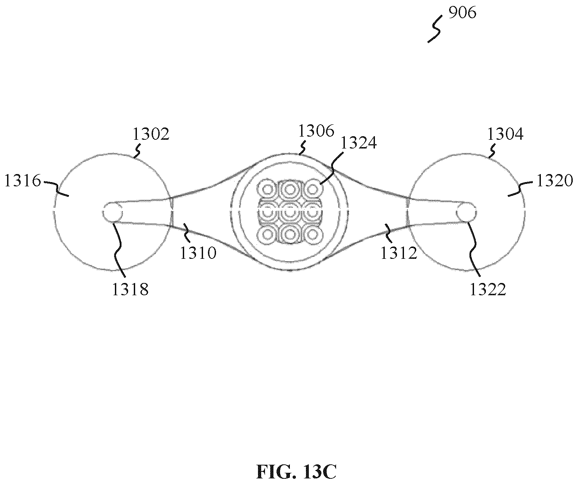

FIGs. 13A-13C show different schematic views of a cell culture module 906 included in the apparatus 900 in accordance with an exemplary embodiment. More specifically, FIG. 13A shows a schematic perspective view of the cell culture module 906, FIG. 13B shows a schematic side view of the cell culture module 906, and FIG. 13C shows a schematic top view of the cell culture module 906. As shown in FIGs. 13A-13C, the cell culture module 906 comprises a first culture medium reservoir 1302, a second culture medium reservoir 1304, a first (apical or top) chamber 1306, a second (basal or bottom) chamber 1308, a first flow channel 1310, a second flow channel 1312, and a cell culture membrane structure 1300. The cell culture membrane structure 1300 comprises a membrane 102, 402 (not shown) comprising a first surface 110 being partially deformed by one or more membrane structure cavities 120 protruding from the first surface (not shown); and one or more through pores 104, 404 formed therein (not shown). The first culture medium reservoir 1302 has a top part with an inlet 1316 for the culture medium and a bottom part with an outlet 1318 for the culture medium. Similarly, the second culture medium reservoir 1304 has a top part with an inlet 1320 for the culture medium and a bottom part with an outlet 1322 for the culture medium. It should be again noted that the present disclosure is not limited to the shown number, arrangement, and interconnection of the constructive elements of the cell culture module 906. In general, the first culture medium reservoir 1302, the second culture medium reservoir 1304, the second chamber 1308, the first flow channel 1310, the second flow channel 1312, and the cell culture membrane structure 1300 may be implemented in the same or similar manner as the first culture medium reservoir 10202, the second culture medium reservoir 10204, the second chamber 1208, the first flow channel 1210, the second flow channel 1212, and the cell culture membrane structure 100, 400, respectively.

At the same time, unlike the first chamber 10206, the first chamber 1306 may be implemented as a hollow tube having a curved or angled bottom. As shown in FIGs 13A-13C, the curved or angled bottom of the first chamber 1306 comprises nine chamber cavities 1324 each provided with one or more chamber holes, so that the first chamber 1306 and the second chamber 1308 are in fluid communication via the through pores of the membrane 1314. It should be obvious to those skilled in the art that the shown number, shape, and arrangement of the chamber cavities 1324 are for illustrative purposes only and should not be construed as any limitation of the present disclosure. For example, there may be a single chamber cavity 1324 with a single chamber hole. In general, the configuration of the chamber cavity or cavities 1324 depends on particular applications (e.g., a number of organoids and/spheroids to be formed on the apical surface of the cell culture membrane structure 1300). In one embodiment, the curved or angled bottom of the first chamber 1306 may be coated, from outside, with a cell adhesion enhancing layer such that the holes of the chamber cavities 1324 remains open. The cell adhesion enhancing layer may be the same as the one mentioned earlier in accordance with the embodiments of the cell culture module 906.

Referring back to FIG. 9, the flow driving unit 904 may be configured to cause the culture medium to flow, in each cell culture module 906 of the cell culture plate 902, between the first and second culture medium reservoirs 10202, 1302 and 10204, 1304. In embodiments, the flow driving unit 904 may be a rocking platform that is configured to periodically rock the cell culture plate 902, thereby providing the culture medium flow. In another embodiments, the flow driving unit 904 may be a pneumatic pump that is configured to supply a compressed gas or a pressurized air to the inlet of each of the first and second culture medium reservoirs 10202, 1302) and 10204, 1304 in each cell culture module 906 of the cell culture plate 902, thereby causing the culture medium flow therein. In yet another embodiments, the flow driving unit 904 may be configured to cause the culture medium flow in each cell culture module 906 of the cell culture plate 902 by pipetting the culture medium from the first culture medium reservoir 10202, 1302 to the second culture medium reservoir 10204, 1304, or vice versa, at regular time intervals or based on a level of the culture medium in each of the reservoirs. Thus, unlike the current microfluidic cell culture devices, the apparatus 900 may be compatible with at least three different flow control methods which are indicated above.

In embodiments, the apparatus 900 may further comprise a variety of electrodes. These electrodes may serve different purposes, and their arrangement inside the apparatus 900 depends on which purpose they are used for. In embodiments, one or more cell culture modules 906 of the cell culture plate 902 may comprise a first electrode arranged in the first chamber 10206, 1306 or the cell culture membrane structure 600, and a second electrode arranged in the second chamber 10208, 1308. In another embodiments, the first electrode may be arranged in the first culture medium reservoir 1020, 1302 and/or the second culture medium reservoir 10204, 1304 in one or more culture modules 106, while the second electrode may be arranged in the first chamber 10206, 1306 or the cell culture membrane structure 600 of the cell culture module(s) 906. These embodiments with the first and second electrodes may be, for example, used to perform real-time Transepithelial/Transendothelial Electrical Resistance (TEER) measurements. In this case, the first and second electrodes may be connected to an external control unit.

Additionally, or alternatively, the apparatus 900 may further comprise two or more third electrodes arranged in the first chamber 10206, 1306 or the cell culture membrane structure 600 of one or more cell culture modules 906. These third electrodes may be arranged on the chamber walls for measuring a fluid level inside the first chamber 10206, 1306 or the cell culture membrane structure 600. In this case, the third electrodes may either be in direct contact with the fluid/culture medium to determine the fluid level by performing conductivity measurements, or be embedded into the chamber walls (e.g., at a distance of 0.5 mm - 2 mm from each other) to determine the fluid level by measuring inductive or capacitive changes in the third electrodes. At the same time, the two third electrodes may be arranged opposite to each other such that one of the two third electrodes is used to issue an ultrasound wave and another of the two third electrodes is used to receive the ultrasound wave passed through the interior of the first chamber 10206, 1306 or the cell culture membrane structure 600 (these ultrasound measurements may be performed just like radar measurements).

Additionally, or alternatively, the apparatus 900 may further comprise two or more fourth electrodes embedded into the membrane 102, 402 of the cell culture membrane structure 100, 400, 600, 1300 of the one or more cell culture modules 906. These fourth electrodes may be used to provide different stimulus signals to cells deposited on the apical and/or basal surface of the membrane.

In embodiments, the first chamber 10206, 1306, the cell culture membrane structure 600, or the second chamber 10208, 1308 of one or more cell culture modules 906 of the cell culture plate 902 may further comprise an oxygen sensor, a pH sensor, a CO₂ sensor, or any combination thereof. These sensors may be used to enable real-time measurements of different parameters crucial for cell behavior.

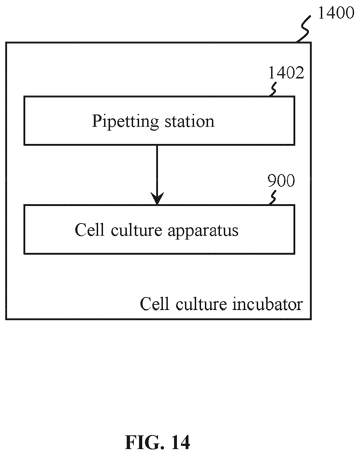

FIG. 14 shows a block diagram of a cell culture incubator 1400 in accordance with an exemplary embodiment. The cell culture incubator 1400 comprises the cell culture apparatus 900, and a pipetting station 1402 coupled to the apparatus 900. More specifically, the pipetting station 1402 is configured to feed the culture medium to each of the first and second culture medium reservoirs 10202, 1302 and 10204, 1304 in each cell culture module 906 of the cell culture plate 902. The pipetting station 1402 is well-known in this technical field, for which reason its description is omitted herein.

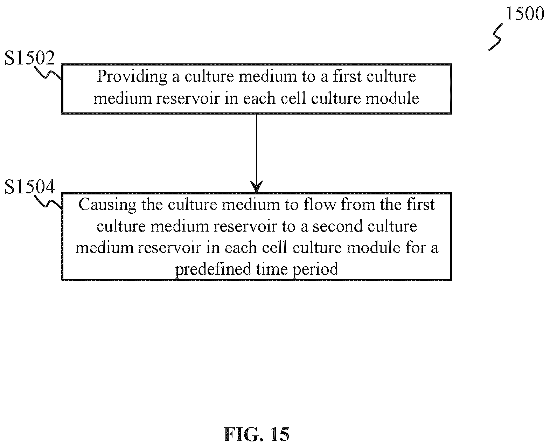

FIG. 15 shows a flowchart of a method 1500 for cell cultivation by using the cell culture apparatus 900 in accordance with an exemplary embodiment. The method 1500 starts with a step S1502, in which a required culture medium is provided, e.g., with the pipetting station 1402, to one of the at least two culture medium reservoirs in each cell culture module of the one or more cell culture modules, e.g., to one of the first and second culture medium reservoirs 10202, 1302 and 10204, 1304 in each cell culture module 906 of the cell culture plate 902. Let us assume that the culture medium is provided to the first culture medium reservoir 10202, 1302. Then, the method 1500 proceeds to a step S1504, in which the culture medium is caused, e.g., by a flow driving unit 904, to flow from said one of the at least two culture medium reservoirs to the rest of the at least two culture medium reservoirs in each cell culture module of the one or more cell culture modules for a predefined time period, the predefined time period being selected based on the culture medium. For example, in S1504, the culture medium is caused, e.g., by a flow driving unit 904, to flow from the first culture medium reservoir 10202, 1302 to the second culture medium reservoir 1020, 1304 in each cell culture module 906 of the cell culture plate 902 for a predefined time period. The predefined time period may be selected based on the culture medium (i.e., a type of cells used therein). In particular, the cell deposition on the basal surface of the membrane 10214 may be achieved if a flow rate is greater than gravity and a continuous flow lasts at least 30 min, preferably 60 min.

In embodiments, in S1504, the causing the culture medium to flow is caused by a flow driving unit. A continuous flow may be achieved which may facilitate the growth and viability of the cells, organoids, and/or spheroids in the apparatus 900.

In embodiments, the method 1500 may comprise, during step S1504, an additional step, in which a different culture medium is fed, e.g., by the pipetting station 1402, via the first chamber 10206, 1306 or the cell culture membrane structure 600 towards the membrane 102, 402 in at least one cell culture module of the one or more cell culture modules 906 of the cell culture plate 902. If the first chamber 10206 or the cell culture membrane structure 600 is used, a cell monolayer is formed on the apical surface of the membrane 102, 402, and this cell monolayer interacts with a cell layer forming on the basal surface of the membrane 102, 402 via the one or more through pores 104, 404 of the membrane 102, 402. If the first chamber 1306 is used, multiple microcavities 1324 (i.e., chamber cavities) are bonded to the membrane 102, 402 of the cell culture membrane structure 1300, for which reason cells from the different culture medium are trapped to form a single cell, spheroid, or organoid in each chamber cavity 1324. In this case, the flow channels 1310 and 1312 may perfuse the culture medium through the membrane 102, 402 of the cell culture membrane structure 1300 to keep the organoids alive. In addition, if the cavity opening 122 of each of the one or more membrane structure cavities 120 of the cell culture membrane structure 100, 400, 600, 1300 is located on the apical surface (i.e., the apical surface is the second surface 112 and the basal surface is the first surface 110) of the membrane 102, 402 of the cell culture membrane structure 100, 400, 600, 1300, the one or more membrane structure cavities 120 may be provided with the another culture medium and a single cell, spheroid, or organoid may be formed in each of the one or more membrane structure cavities 120. Using this arrangement, the apparatus may even broaden the possibilities of cell behavior studies of the cell, spheroid, or organoid. If on the other hand the cavity opening 122 of each of the one or more membrane structure cavities 120 of the cell culture membrane structure 100, 400, 600, 1300 is located on the basal surface (i.e., the basal surface is the second surface 112 and the apical surface is the first surface 110) of the membrane 102, 402 of the cell culture membrane structure 100, 400, 600, 1300, the one or more membrane structure cavities 120 (and the basal surface) may be provided with the culture medium and the apical surface of the membrane may be provided with the another culture medium. A single cell, spheroid, or organoid may be formed in each of the one or more membrane structure cavities 120, each of the one or more membrane structure cavities 120 having a cavity opening 122 on the basal surface of the membrane 102, 402 (i.e., each cavity opening 122 being on the second chamber side of the membrane). Using this arrangement, the apparatus may even broaden the possibilities of cell behavior studies of the cell, spheroid, or organoid.

In embodiments, the step S1504 of the method 1500 may be performed by applying a positive pressure to the first and second culture medium reservoirs 10210, 1310 and 10212, 1312 in each cell culture module 906 for the predefined time period (e.g., selected from 1 - 120 min, or 30 min, or 60 min, or 90 min). In another embodiments, the step S1504 of the method 1500 may be performed by applying a positive pressure to said one of the at least two culture medium reservoirs 10202, 1302, 10204, 1304 in each cell culture module of the one or more cell culture modules 906 for the predefined time period (e.g., selected from 1 - 120 min, or 30 min, or 60 min, or 90 min), while capping the rest of the at least two culture medium reservoirs 10202, 1302, 10204, 1304. I.e., for example, applying a positive pressure to the first culture medium reservoir 10202, 1302 in each cell culture module of the one or more cell culture modules 906 for the predefined time period (e.g., selected from 1 - 120 min, or 30 min, or 60 min, or 90 min), while capping the second culture medium reservoir 10204, 1304, or vice versa. In yet another embodiments, wherein the at least two culture medium reservoirs in each cell culture module of the one or more cell culture modules comprises a first culture medium reservoir 10202, 1302 and a second culture medium reservoir 10204, 1304, the step S1504 of the method 1500 may be performed by: applying a first positive pressure to the first culture medium reservoir 1020, 1302 in each cell culture module 906 for the predefined time interval, while capping the second culture medium reservoir 10204, 1304; and applying a second positive pressure to the second culture medium reservoir 10204, 1304 in each cell culture module 906 for the predefined time interval, while capping the first culture medium reservoir 10202, 1302. The selection of each of the above-indicated embodiments of the step S1504 depends on particular applications.

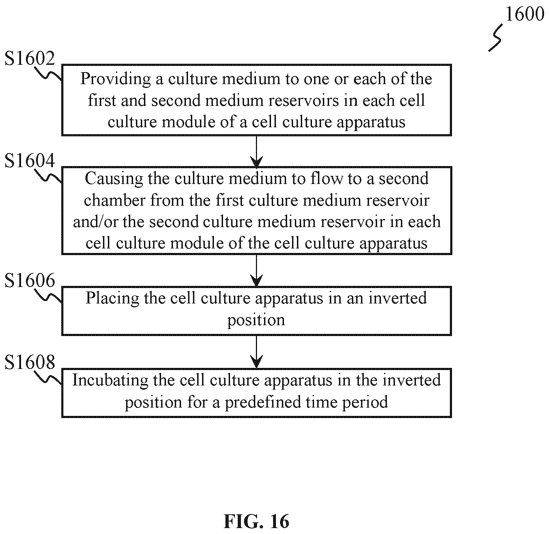

FIG. 16 shows a flowchart of a method 1600 for cell cultivation by using the cell culture apparatus 900 in accordance with an exemplary embodiment. The method 1600 comprises a step S1602, in which a culture medium is provided to one or more of the at least two culture medium reservoirs 10202, 1302 and 10204, 1304 in each cell culture module 906 of the one or more cell culture modules. The culture medium may be provided to each of the first and second medium reservoirs 10202, 1302 and 10204, 1304 in each cell culture module 906. Then, the method 1600 proceeds to a step S1604, in which the culture medium is caused to flow to the second chamber 10208, 1308 from said one or more of the at least two culture medium reservoirs 10202, 1302 and/or 10204, 1304 in each cell culture module 906 of the one or more cell culture modules. I.e., for example, the culture medium is caused to flow to the second chamber 10208, 1308 from the first medium culture reservoir 10202, 1302 and/or the second medium reservoir 10204, 1304 in each cell culture module 906 (of the cell culture plate 902). After that, the method 1600 goes on to a step S1606, in which the cell culture apparatus 900 is placed in an inverted position. Next, the method 1600 proceeds to a step S1608, in which the cell culture apparatus 900 is incubated in the inverted position for a predefined time period. The predefined time period is again selected based on the culture medium. In embodiments, the culture medium is caused to flow by the flow driving unit 904.

In embodiments, similar to the method 1500, the method 1600 further comprises, during the step S1604, an additional step, in which a different culture medium is fed, e.g., by the pipetting station 1402, via the first chamber 10206 (1306) or the cell culture membrane structure 600 towards the membrane 102, 402 in at least one cell culture module 906 of the one or more cell culture modules (of the cell culture plate 902). If the first chamber 10206 or the cell culture membrane structure 600 is used, a cell monolayer is formed on the apical surface of the membrane 102, 402. If the first chamber 1306 is used, a single cell, spheroid, or organoid is formed in each chamber cavity 1324 of the first chamber 1306. The culture medium perfused through the flow channels 10210, 1310 and 10212, 1312 may comprise human brain vascular endothelial cells, while the different culture medium fed to the first chamber 10206, 1305 or the cell culture membrane structure 600 may comprise human astrocytes. By using these types of cells, it is possible to mimic an artificial blood-brain barrier (BBB). In addition, if the cavity opening 122 of each of the one or more membrane structure cavities 120 of the cell culture membrane structure 100, 400, 600, 1300 is located on the apical surface (i.e., the apical surface is the second surface 112 and the basal surface is the first surface 110) of the membrane 102, 402 of the cell culture membrane structure 100, 400, 600, 1300, the one or more membrane structure cavities 120 may be provided with the another culture medium and a single cell, spheroid, or organoid may be formed in each of the one or more membrane structure cavities 120. For example, if the culture medium perfused through the flow channels 10210, 1310 and 10212, 1312 comprises human brain vascular endothelial cells, while the another culture medium fed to the first chamber 10206, 1305 or the cell culture membrane structure 1300 comprises human astrocytes, a single cell, spheroid, or organoid may be formed from the human brain vascular endothelial cells in each of the one or more membrane structure cavities 120, even though the first chamber 10206 and the cell culture membrane structure 600 do not comprise one or more chamber cavities 1324. By using these types of cells, it is possible to mimic an artificial blood-brain barrier (BBB). If on the other hand the cavity opening 122 of each of the one or more membrane structure cavities 120 of the cell culture membrane structure 100, 400, 600, 1300 is located on the basal surface (i.e., the basal surface is the second surface 112 and the apical surface is the first surface 110) of the membrane 102, 402 of the cell culture membrane structure 100, 400, 600, 1300, the one or more membrane structure cavities 120 (and the basal surface) may be provided with the culture medium and the apical surface of the membrane may be provided with the another culture medium. A single cell, spheroid, or organoid may be formed in each of the one or more membrane structure cavities 120, each of the one or more membrane structure cavities 120 having a cavity opening 122 on the basal surface of the membrane 102, 402 (i.e., each cavity opening 122 being on the second chamber side of the membrane). For example, if the culture medium perfused through the flow channels 10210, 1310 and 10212, 1312 comprises human astrocytes, while the another culture medium fed to the first chamber 10206, 1305 or the cell culture membrane structure 1300 comprises human brain vascular endothelial cells, a single cell, spheroid, or organoid may be formed from the human astrocytes in each of the one or more membrane structure cavities 120, even though the first chamber 10206 and the cell culture membrane structure 600 do not comprise one or more chamber cavities 1324.

Although the exemplary embodiments of the present disclosure are described herein, it should be noted that any various changes and modifications could be made in the embodiments of the present disclosure, without departing from the scope of legal protection which is defined by the appended claims. In the appended claims, the word "comprising" does not exclude other elements, steps or operations, and the indefinite article "a" or "an" does not exclude a plurality. The mere fact that certain measures are recited in mutually different dependent claims does not indicate that a combination of these measures cannot be used to advantage.

## Claims

1. A cell culture membrane structure (100, 400, 600, 1300) comprising:
- a membrane (102, 402) made of a membrane material, the membrane comprising:
- a first surface (110) being partially deformed by one or more membrane structure cavities (120) protruding from the first surface, and
- one or more through pores (104, 404) formed therein;
wherein the membrane material is selected based on at least one type of cells to be cultured on the membrane, wherein each of the one or more membrane structure cavities has a cavity opening (122) being each independently 50 - 3000 µm wide.

2. The cell culture membrane structure as claimed in the preceding claim, wherein each of the one or more membrane structure cavities has an inner width (126) being in the range of 50 - 3000 µm.

3. The cell culture membrane structure as claimed in any of the preceding claims, wherein the cell culture membrane structure further comprises at least one coating layer (106, 108, 406, 408) being made of a coating material, the at least one coating layer provided on the membrane (102, 402) such that the one or more membrane structure cavities (120) and the one or more through pores (104, 404) of the membrane (102, 402) remain open, wherein the membrane material and the coating material are selected based on at least one type of cells to be cultured on the membrane and/or on the at least one coating layer.

4. The cell culture membrane structure as claimed in claim 3, wherein the at least one coating layer (106, 108, 406, 408) comprises one first coating layer (106, 406) provided on a second surface (112) of the membrane and one second coating layer (108, 408) provided on the first surface (110) of the membrane.

5. The cell culture membrane structure (600) as claimed in any of claims 1 - 4, wherein the cell culture membrane structure further comprises an insert wall (660) attached to the cell culture membrane structure, an inner insert cavity (608), and an insert opening (670); wherein the inner insert cavity is surrounded by the insert opening, the membrane, and the insert wall; the insert wall extending from the membrane to the insert opening; and the inner insert cavity is located between the insert opening and the membrane.

6. A cell culture plate (700) comprising:
a housing (702) encompassing one or more wells (704), each well being arranged to retain a cell culture media, and
one or more cell culture membrane structures (600) as defined in claim 5 each being a replaceable insert configured to be immersed into the cell culture media such that each of the inner insert cavities of each of the replaceable inserts is in flow communication with one well of the housing via the through pores.

7. A cell culture apparatus (900) comprising:
one or more cell culture modules (906), each cell culture module of the one or more cell culture modules comprising:
- at least two culture medium reservoirs (10202, 10204, 1302, 1304) each having a top part and a bottom part, the top part having an inlet (10216, 10220, 1316, 1320) for a culture medium and the bottom part having an outlet (10218, 10222, 1318, 1322) for the culture medium;
- a first chamber (10206, 1306) configured to be arranged between the at least two culture medium reservoirs such that the first chamber and the at least two culture medium reservoirs are aligned with each other in a first direction, and
- a cell culture membrane structure (100, 400, 1300) as defined in any of claims 1 - 4 configured to be arranged between the first chamber and a second chamber (10208, 1308) such that the first chamber and the second chamber are in flow communication with each other via the through pores; or
- a cell culture membrane structure (600) as defined in claim 5 configured to be arranged between the at least two culture medium reservoirs such that the cell culture membrane structure (600) and the at least two culture medium reservoirs are aligned with each other in a first direction, the membrane (102, 402) of the cell culture membrane structure (600) being arranged between the inner insert cavity and a second chamber (10208) such that the inner insert cavity and the second chamber are in flow communication with each other via the through pores;
wherein the second chamber being either arranged under and aligned with the first chamber, or arranged under and aligned with the cell culture membrane structure (600) as defined in claim 5, in a second direction, the second direction having an angle being more than 0 degrees but 90 degrees or less in respect to the first direction, the second chamber having a bottom part having at least two lateral holes; and
- at least two flow channels (10210, 10212, 1310, 1312), each of the at least two flow channels being configured to pass a fluid or cell culture media therethrough, and each of the at least two flow channels connecting the outlet of the bottom part of one of the at least two culture medium reservoirs to one of the at least two lateral holes of the bottom part of the second chamber.

8. The cell culture apparatus as claimed in claim 7, wherein the apparatus comprises the cell culture membrane structure (600) as defined in claim 5, and the apparatus further comprises a first chamber (10206) configured to be arranged between the at least two culture medium reservoirs such that the first chamber and the at least two culture medium reservoirs are aligned with each other in the first direction, wherein the cell culture membrane structure (600) as defined in claim 5 being configured to be detachably inserted in the first chamber, the second chamber (10208) being arranged under and aligned with the cell culture membrane structure (600) as defined in claim 5 in the second direction.

9. The cell culture apparatus as claimed in any of claims 7 - 8, wherein the apparatus comprises a plurality of cell culture modules arranged adjacent to each other.

10. The cell culture apparatus as claimed in any of claims 7 - 9, wherein the first chamber in at least one cell culture module of the one or more cell culture modules is implemented as a bottomless hollow tube.

11. The cell culture apparatus as claimed in any of claims 7 - 9, wherein the first chamber (1306) in at least one cell culture module of the one or more cell culture modules is implemented as a hollow tube having a curved or angled bottom, the curved or angled bottom having at least one first chamber cavity (1324) and at least one first chamber hole formed in each of the at least one first chamber cavity.

12. The cell culture apparatus of claim 11, wherein the curved or angled bottom is coated, from outside, with a cell adhesion enhancing layer such that the at least one first chamber hole of the at least one first chamber cavity remains open, the cell adhesion enhancing layer being made of a hydrophilic material.

13. The cell culture apparatus as claimed in any of claims 7 - 12, wherein each of the at least two culture medium reservoirs in at least one cell culture module of the one or more cell culture modules is implemented as a hollow tube.

14. The cell culture apparatus as claimed in any of claims 7 - 13, wherein at least one cell culture module of the one or more cell culture modules further comprises at least two electrodes, wherein each of the at least two electrodes is independently arranged in the first chamber, the cell culture membrane structure (600) as defined in claim 5, the second chamber, in one or more of the at least two culture medium reservoirs, or the membrane, or any combinations thereof.

15. A cell culture incubator (1400) comprising:
the cell culture apparatus (900) as defined in any of claims 7 - 14; and
a pipetting station (1402) configured to feed the culture medium to each of the at least two culture medium reservoirs in each cell culture module of the one or more cell culture modules.

16. A method for producing a cell culture membrane structure, comprising:
- providing a membrane (102, 402) made of a membrane material,
- applying at least one coating layer (106, 108, 406, 408) on the membrane (102, 402) in a continuous manner, the at least one coating layer (106, 108, 406, 408) being made of a coating material; and
- forming through pores (104, 404) coming through the at least one coating layer (106, 108, 406, 408) and the membrane (102, 402);
wherein the membrane material and the coating material are selected based on at least one type of cells to be cultured on the at least one coating layer,
wherein the membrane made of a membrane material comprises a first surface (110) being partially deformed by one or more membrane structure cavities (120) protruding from the first surface (110); or
wherein the method further comprises, before applying at least one coating layer, before forming through pores, or after forming through pores, deforming the membrane, thereby obtaining one or more membrane structure cavities (120) protruding from a first surface of the membrane (110).

17. A method for cell cultivation by using the cell culture apparatus as defined in any of claims 7 - 14, the method comprising:
(a) providing the culture medium to one of the at least two culture medium reservoirs in each cell culture module of the one or more cell culture modules; and
(b) causing the culture medium to flow from said one of the at least two culture medium reservoirs to the rest of the at least two culture medium reservoirs in each cell culture module of the one or more cell culture modules for a predefined time period, the predefined time period being selected based on the culture medium.

18. A method for cell cultivation by using the cell culture apparatus as defined in any of claims 7 - 14, the method comprising:
(a) providing the culture medium to one or more of the at least two culture medium reservoirs in each cell culture module of the one or more cell culture modules;
(b) causing the culture medium to flow to the second chamber from said one or more of the at least two culture medium reservoirs in each cell culture module of the one or more cell culture modules;
(c) placing the cell culture apparatus in an inverted position; and
(d) incubating the cell culture apparatus in the inverted position for a predefined time period, the predefined time period being selected based on the culture medium.

19. The method as claimed in any of claims 17 - 18, further comprising, during (b), feeding another culture medium via the first chamber or the cell culture membrane structure (600) as defined in claim 5 towards the membrane in at least one cell culture module of the one or more cell culture modules.

20. The method as claimed in any of claims 17 - 19, wherein the culture medium comprises brain vascular endothelial cells and said another culture medium comprises astrocytes.

## Patentansprüche

1. Zellkulturmembranstruktur (100, 400, 600, 1300), umfassend:
- eine Membran (102, 402), die aus einem Membranmaterial hergestellt ist, wobei die Membran folgendes umfasst:
- eine erste Oberfläche (110), die teilweise durch eine oder mehrere Membranstrukturkavitäten (120) deformiert ist, die aus der ersten Oberfläche hervorstehen, und
- eine oder mehrere darin gebildete Durchgangsporen (104, 404);
wobei das Membranmaterial basierend auf mindestens einem Zelltyp ausgewählt ist, der auf der Membran kultiviert werden soll, wobei jede der einen oder mehreren Membranstrukturkavitäten eine Kavitätsöffnung (122) aufweist, die jeweils unabhängig voneinander 50 - 3000µm breit sind.

2. Zellkulturmembranstruktur gemäß dem vorhergehenden Anspruch, wobei jede der einen oder mehreren Membranstrukturkavitäten eine innere Breite (126) in dem Bereich von 50 - 3000µm aufweist.

3. Zellkulturmembranstruktur gemäß einem der vorhergehenden Ansprüche, wobei die Zellkulturmembranstruktur ferner mindestens eine Beschichtungsschicht (106, 108, 406, 408) umfasst, die aus einem Beschichtungsmaterial hergestellt ist, wobei die mindestens eine Beschichtungsschicht auf der Membran (102, 402) bereitgestellt ist, derart, dass die eine oder mehreren Membranstrukturkavitäten (120) und die eine oder mehreren Durchgangsporen (104, 404) der Membran (102, 402) offen bleiben, wobei das Membranmaterial und das Beschichtungsmaterial basierend auf mindestens einem Zelltyp ausgewählt sind, der auf der Membran und/oder auf der mindestens einen Beschichtungsschicht kultiviert werden soll.

4. Zellkulturmembranstruktur gemäß Anspruch 3, wobei die mindestens eine Beschichtungsschicht (106, 108, 406, 408) eine erste Beschichtungsschicht (106, 406), die auf einer zweiten Oberfläche (112) der Membran bereitgestellt ist, und eine zweite Beschichtungsschicht (108, 408), die auf der ersten Oberfläche (110) der Membran bereitgestellt ist, umfasst.

5. Zellkulturmembranstruktur (600) gemäß einem der Ansprüche 1 - 4, wobei die Zellkulturmembranstruktur ferner eine Einsatzwand (660), die an der Zellkulturmembranstruktur angebracht ist, eine innere Einsatzkavität (608), und eine Einsatzöffnung (670) umfasst; wobei die innere Einsatzkavität von der Einsatzöffnung, der Membran, und der Einsatzwand umgeben ist; wobei sich die Einsatzwand von der Membran bis zur Einsatzöffnung erstreckt; und wobei die innere Einsatzkavität zwischen der Einsatzöffnung und der Membran lokalisiert ist.

6. Zellkulturplatte (700), umfassend:
ein Gehäuse (702), das eine oder mehrere Vertiefungen (704) umfasst, wobei jede Vertiefung dazu ausgelegt ist, ein Zellkulturmedium aufzunehmen, und
eine oder mehrere Zellkulturmembranstrukturen (600), wie in Anspruch 5 definiert, die jeweils einen austauschbaren Einsatz darstellen, der dazu konfiguriert ist, in das Zellkulturmedium eingesetzt zu werden, derart, dass jede der inneren Einsatzkavitäten jedes der austauschbaren Einsätze über die Durchgangsporen in Strömungsverbindung mit einer Vertiefung des Gehäuses ist.

7. Zellkulturvorrichtung (900), umfassend:
ein oder mehrere Zellkulturmodule (906), wobei jedes Zellkulturmodul der einen oder mehreren Zellkulturmodule umfasst:
- mindestens zwei Kulturmediumreservoirs (10202, 10204, 1302, 1304), die jeweils einen oberen Teil und einen unteren Teil aufweisen, wobei der obere Teil einen Einlass (10216, 10220, 1316, 1320) für ein Kulturmedium und der untere Teil einen Auslass (10218, 10222, 1318, 1322) für das Kulturmedium aufweist;
- eine erste Kammer (10206, 1306), die dazu konfiguriert ist, zwischen den mindestens zwei Kulturmediumreservoirs angeordnet zu sein, derart, dass die erste Kammer und die mindestens zwei Kulturmediumreservoirs in einer ersten Richtung miteinander ausgerichtet sind, und
- eine Zellkulturmembranstruktur (100, 400, 1300), wie in einem der Ansprüche 1 bis 4 definiert, die dazu konfiguriert ist, zwischen der ersten Kammer und einer zweiten Kammer (10208, 1308) angeordnet zu sein, derart, dass die erste Kammer und die zweite Kammer über die Durchgangsporen in Strömungsverbindung miteinander sind; oder
- eine Zellkulturmembranstruktur (600), wie in Anspruch 5 definiert, die dazu konfiguriert ist, zwischen den mindestens zwei Kulturmediumreservoirs angeordnet zu sein, derart, dass die Zellkulturmembranstruktur (600) und die mindestens zwei Kulturmediumreservoirs in einer ersten Richtung miteinander ausgerichtet sind, wobei die Membran (102, 402) der Zellkulturmembranstruktur (600) zwischen der inneren Einsatzkavität und einer zweiten Kammer (10208) angeordnet ist, derart, dass die innere Einsatzkavität und die zweite Kammer über die Durchgangsporen in Strömungsverbindung miteinander sind;
wobei die zweite Kammer entweder unterhalb der ersten Kammer angeordnet und mit dieser ausgerichtet ist, oder unterhalb der Zellkulturmembranstruktur (600), wie in Anspruch 5 definiert, angeordnet und mit dieser ausgerichtet ist, in einer zweiten Richtung, wobei die zweite Richtung einen Winkel von mehr als 0 Grad, aber 90 Grad oder weniger in Bezug auf die erste Richtung aufweist, wobei die zweite Kammer einen unteren Teil mit mindestens zwei lateralen Löchern aufweist; und
- mindestens zwei Strömungskanäle (10210, 10212, 1310, 1312), wobei jeder der mindestens zwei Strömungskanäle dazu konfiguriert ist, ein Fluid oder ein Zellkulturmedium durch ihn hindurchfließen zu lassen, und wobei jeder der mindestens zwei Strömungskanäle den Auslass des unteren Teils eines der mindestens zwei Kulturmediumreservoirs mit einem der mindestens zwei lateralen Löcher des unteren Teils der zweiten Kammer verbindet.

8. Zellkulturvorrichtung gemäß Anspruch 7, wobei die Vorrichtung die Zellkulturmembranstruktur (600), wie in Anspruch 5 definiert, umfasst und die Vorrichtung ferner eine erste Kammer (10206) umfasst, die dazu konfiguriert ist, zwischen den mindestens zwei Kulturmediumreservoirs angeordnet zu werden, derart, dass die erste Kammer und die mindestens zwei Kulturmediumreservoirs in der ersten Richtung miteinander ausgerichtet sind, wobei die Zellkulturmembranstruktur (600), wie in Anspruch 5 definiert, so konfiguriert ist, dass sie lösbar in die erste Kammer eingesetzt werden kann, wobei die zweite Kammer (10208) unterhalb der Zellkulturmembranstruktur (600), wie in Anspruch 5 definiert, in der zweiten Richtung angeordnet und mit dieser ausgerichtet ist.

9. Zellkulturvorrichtung gemäß einem der Ansprüche 7-8, wobei die Vorrichtung eine Mehrzahl von Zellkulturmodulen umfasst, die benachbart zueinander angeordnet sind.

10. Zellkulturvorrichtung gemäß einem der Ansprüche 7-9, wobei die erste Kammer in mindestens einem Zellkulturmodul des einen oder der mehreren Zellkulturmodule als bodenloses hohles Rohr implementiert ist.

11. Zellkulturvorrichtung gemäß einem der Ansprüche 7-9, wobei die erste Kammer (1306) in mindestens einem Zellkulturmodul des einen oder der mehreren Zellkulturmodule als hohles Rohr mit einem gekrümmten oder abgewinkelten Boden implementiert ist, wobei der gekrümmte oder abgewinkelte Boden mindestens eine erste Kammerkavität (1324) und mindestens ein erstes Kammerloch aufweist, das in jeder der mindestens einen ersten Kammerkavität gebildet ist.

12. Zellkulturvorrichtung gemäß Anspruch 11, wobei der gekrümmte oder abgewinkelte Boden von außen mit einer Zelladhäsionsverbesserungsschicht beschichtet ist, derart, dass das mindestens eine erste Kammerloch der mindestens einen ersten Kammerkavität offen bleibt, wobei die Zelladhäsionsverbesserungsschicht aus einem hydrophilen Material hergestellt ist.

13. Zellkulturvorrichtung gemäß einem der Ansprüche 7-12, wobei jedes der mindestens zwei Kulturmediumreservoirs in mindestens einem Zellkulturmodul des einen oder der mehreren Zellkulturmodule als hohles Rohr implementiert ist.

14. Zellkulturvorrichtung gemäß einem der Ansprüche 7-13, wobei mindestens ein Zellkulturmodul des einen oder der mehreren Zellkulturmodule ferner mindestens zwei Elektroden umfasst, wobei jede der mindestens zwei Elektroden unabhängig voneinander in der ersten Kammer, der Zellkulturmembranstruktur (600), wie in Anspruch 5 definiert, der zweiten Kammer, in einem oder mehreren der mindestens zwei Kulturmediumreservoirs oder der Membran, oder einer beliebigen Kombination davon angeordnet ist.

15. Zellkulturinkubator (1400) umfassend:
die Zellkulturvorrichtung (900), wie in einem der Ansprüche 7 bis 14 definiert; und
eine Pipettierstation (1402), die dazu konfiguriert ist, das Kulturmedium zu jedem der mindestens zwei Kulturmediumreservoirs in jedem Zellkulturmodul des einen oder der mehreren Zellkulturmodule zuzuführen.

16. Verfahren zum Herstellen einer Zellkulturmembranstruktur, umfassend:
- Bereitstellen einer Membran (102, 402), die aus einem Membranmaterial hergestellt ist,
- Aufbringen mindestens einer Beschichtungsschicht (106, 108, 406, 408) auf die Membran (102, 402) in einer kontinuierlichen Weise, wobei die mindestens eine Beschichtungsschicht (106, 108, 406, 408) aus einem Beschichtungsmaterial hergestellt wird; und
- Bilden von Durchgangsporen (104, 404), die durch die mindestens eine Beschichtungsschicht (106, 108, 406, 408) und die Membran (102, 402) hindurchgehen;
wobei das Membranmaterial und das Beschichtungsmaterial basierend auf mindestens einem Zelltyp ausgewählt sind, der auf der mindestens einen Beschichtungsschicht kultiviert werden soll,
wobei die Membran aus einem Membranmaterial hergestellt ist, das eine erste Oberfläche (110) umfasst, die durch eine oder mehrere Membranstrukturkavitäten (120), die aus der ersten Oberfläche (110) hervorstehen, teilweise deformiert ist; oder
wobei das Verfahren ferner umfasst, dass vor dem Aufbringen mindestens einer Beschichtungsschicht, vor dem Bilden von Durchgangsporen, oder nach dem Bilden von Durchgangsporen, die Membran deformiert wird, wodurch ein oder mehrere Membranstrukturkavitäten (120) erhalten werden, die von der ersten Oberfläche der Membran (110) hervorstehen.

17. Verfahren zur Zellkultivierung unter Verwendung der Zellkulturvorrichtung, wie in einem der Ansprüche 7-14 definiert, wobei das Verfahren umfasst:
a) Bereitstellen des Kulturmediums an eines der mindestens zwei Kulturmediumreservoirs in jedem Zellkulturmodul des einen oder der mehreren Zellkulturmodule; und
b) Verursachen, dass das Kulturmedium für eine vordefinierte Zeitperiode von dem einen der mindestens zwei Kulturmediumreservoirs zu den übrigen der mindestens zwei Kulturmediumreservoirs in jedem Zellkulturmodul des einen oder der mehreren Zellkulturmodule fließt, wobei die vordefinierte Zeitperiode basierend auf dem Kulturmedium ausgewählt wird.

18. Verfahren zur Zellkultivierung unter Verwendung der Zellkulturvorrichtung, wie in einem der Ansprüche 7-14 definiert, wobei das Verfahren umfasst:
a) Bereitstellen des Kulturmediums an eines oder mehrere der mindestens zwei Kulturmediumreservoirs in jedem Zellkulturmodul des einen oder der mehreren Zellkulturmodule;
b) Verursachen, dass das Kulturmedium aus dem einen oder den mehreren der mindestens zwei Kulturmediumreservoirs in jedem Zellkulturmodul des einen oder der mehreren Zellkulturmodule in die zweite Kammer fließt;
c) Platzieren der Zellkulturvorrichtung in einer invertierten Position; und
d) Inkubieren der Zellkulturvorrichtung in der invertierten Position für eine vordefinierte Zeitperiode, wobei die vordefinierte Zeitperiode basierend auf dem Kulturmedium ausgewählt wird.

19. Verfahren gemäß einem der Ansprüche 17 - 18, das ferner während (b) das Zuführen eines weiteren Kulturmediums über die erste Kammer oder die Zellkulturmembranstruktur (600), wie in Anspruch 5 definiert, in Richtung der Membran in mindestens ein Zellkulturmodul des einen oder der mehreren Zellkulturmodule umfasst.

20. Verfahren gemäß einem der Ansprüche 17 - 19, wobei das Kulturmedium Hirngefäßendothelzellen umfasst und das andere Kulturmedium Astrozyten umfasst.

## Revendications

1. Structure de membrane de culture cellulaire (100, 400, 600, 1300) comprenant :
- une membrane (102, 402) faite en un matériau membranaire, la membrane comprenant :
- une première surface (110) qui est partiellement déformée par une ou plusieurs cavités (120) de structure de membrane faisant saillie depuis la première surface, et
- un ou plusieurs pores traversants (104, 404) formés dans celle-ci ; dans laquelle le matériau membranaire est sélectionné sur la base d'au moins un type de cellules à cultiver sur la membrane, dans laquelle chacune des une ou plusieurs cavités de structure de membrane présente une ouverture (122) de cavité, chacune faisant indépendamment 50 à 3000 µm de large.

2. Structure de membrane de culture cellulaire selon la revendication précédente, dans laquelle chacune des une ou plusieurs cavités de structure de membrane présente une largeur interne (126) qui est dans la plage allant de 50 à 3000 µm.

3. Structure de membrane de culture cellulaire selon l'une quelconque des revendications précédentes, dans laquelle la structure de membrane de culture cellulaire comprend en outre au moins une couche de revêtement (106, 108, 406, 408) qui est faite en un matériau de revêtement, ladite au moins une couche de revêtement étant disposée sur la membrane (102, 402) de telle sorte que les une ou plusieurs cavités (120) de structure de membrane et lesdits un ou plusieurs pores traversants (104, 404) de la membrane (102, 402) restent ouverts, dans laquelle le matériau membranaire et le matériau de revêtement sont sélectionnés sur la base d'au moins un type de cellules à cultiver sur la membrane et/ou sur ladite au moins une couche de revêtement.

4. Structure de membrane de culture cellulaire selon la revendication 3, dans laquelle ladite au moins une couche de revêtement (106, 108, 406, 408) comprend une première couche de revêtement (106, 406) disposée sur une seconde surface (112) de la membrane et une seconde couche de revêtement (108, 408) disposée sur la première surface (110) de la membrane.

5. Structure de membrane de culture cellulaire (600) selon l'une quelconque des revendications 1 à 4, dans laquelle la structure de membrane de culture cellulaire comprend en outre une paroi d'insert (660) fixée à la structure de membrane de culture cellulaire, une cavité d'insert interne (608) et une ouverture d'insert (670) ; dans laquelle la cavité d'insert interne est entourée par l'ouverture d'insert, la membrane et la paroi d'insert ; la paroi d'insert s'étendant depuis la membrane jusqu'à l'ouverture d'insert ; et la cavité d'insert interne est située entre l'ouverture d'insert et la membrane.

6. Plaque de culture cellulaire (700) comprenant :
un boîtier (702) englobant un ou plusieurs puits (704), chaque puits étant conçu pour contenir un milieu de culture cellulaire, et
une ou plusieurs structures de membrane de culture cellulaire (600) telles que définies dans la revendication 5 qui sont chacune un insert remplaçable configuré pour être immergé dans le milieu de culture cellulaire de telle sorte que chacune des cavités d'insert interne de chacun des inserts remplaçables est en communication fluidique avec un puits du boîtier via les pores traversants.

7. Appareil de culture cellulaire (900) comprenant :
un ou plusieurs modules de culture cellulaire (906), chaque module de culture cellulaire desdits un ou plusieurs modules de culture cellulaire comprenant :
- au moins deux réservoirs de milieu de culture (10202, 10204, 1302, 1304) présentant chacun une partie supérieure et une partie inférieure, la partie supérieure présentant une entrée (10216, 10220, 1316, 1320) pour un milieu de culture et la partie inférieure présentant une sortie (10218, 10222, 1318, 1322) pour le milieu de culture ;
- une première chambre (10206, 1306) configurée pour être agencée entre lesdits au moins deux réservoirs de milieu de culture de telle sorte que la première chambre et lesdits au moins deux réservoirs de milieu de culture sont alignés l'un par rapport à l'autre dans une première direction, et
- une structure de membrane de culture cellulaire (100, 400, 1300) telle que définie dans l'une quelconque des revendications 1 à 4 configurée pour être agencée entre la première chambre et une seconde chambre (10208, 1308) de telle sorte que la première chambre et la seconde chambre sont en communication fluidique l'une avec l'autre via les pores traversants ; ou
- une structure de membrane de culture cellulaire (600) telle que définie dans la revendication 5 configurée pour être agencée entre lesdits au moins deux réservoirs de milieu de culture de telle sorte que la structure de membrane de culture cellulaire (600) et lesdits au moins deux réservoirs de milieu de culture sont alignés l'un par rapport à l'autre dans une première direction, la membrane (102, 402) de la structure de membrane de culture cellulaire (600) étant agencée entre la cavité d'insert interne et une seconde chambre (10208) de telle sorte que la cavité d'insert interne et la seconde chambre sont en communication fluidique l'une avec l'autre via les pores traversants ;
dans lequel la seconde chambre est agencée sous la première chambre et alignée avec cette dernière, ou agencée sous la structure de membrane de culture cellulaire (600) telle que définie dans la revendication 5 et alignée avec cette structure, dans une seconde direction, la seconde direction présentant un angle supérieur à 0 degré, mais inférieur ou égal à 90 degrés, par rapport à la première direction, la seconde chambre présentant une partie inférieure présentant au moins deux trous latéraux ;
- au moins deux canaux d'écoulement (10210, 10212, 1310, 1312), chacun des au moins deux canaux d'écoulement étant configuré pour faire passer à travers lui un fluide ou milieu de culture cellulaire, et chacun des au moins deux canaux d'écoulement raccordant la sortie de la partie inférieure de l'un desdits au moins deux réservoirs de milieu de culture à l'un des au moins deux trous latéraux de la partie inférieure de la seconde chambre.

8. Appareil de culture cellulaire selon la revendication 7, dans lequel l'appareil comprend la structure de membrane de culture cellulaire (600) telle que définie dans la revendication 5, et l'appareil comprend en outre une première chambre (10206) configurée pour être agencée entre lesdits au moins deux réservoirs de milieu de culture de telle sorte que la première chambre et lesdits au moins deux réservoirs de milieu de culture sont alignés l'un par rapport à l'autre dans une première direction,
dans lequel la structure de membrane de culture cellulaire (600) telle que définie dans la revendication 5 est configurée pour être insérée de manière amovible dans la première chambre, la seconde chambre (10208) étant agencée sous la structure de membrane de culture cellulaire (600) telle que définie dans la revendication 5 et alignée sous cette structure dans la seconde direction.

9. Appareil de culture cellulaire selon l'une quelconque des revendications 7 à 8, dans lequel l'appareil comprend une pluralité de modules de culture cellulaire agencés de manière adjacente les uns aux autres.

10. Appareil de culture cellulaire selon l'une quelconque des revendications 7 à 9, dans lequel la première chambre dans au moins un module de culture cellulaire desdits un ou plusieurs modules de culture cellulaire est mise en œuvre sous la forme d'un tube creux sans fond.

11. Appareil de culture cellulaire selon l'une quelconque des revendications 7 à 9, dans lequel la première chambre (1306) dans au moins un module de culture cellulaire desdits un ou plusieurs modules de culture cellulaire est mise en œuvre sous la forme d'un tube creux présentant un fond incurvé ou incliné, le fond incurvé ou incliné présentant au moins une première cavité (1324) de chambre et au moins un premier trou de chambre formé dans chacune de ladite au moins une première cavité de chambre.

12. Appareil de culture cellulaire selon la revendication 11, dans lequel le fond incurvé ou incliné est recouvert, depuis l'extérieur, d'une couche d'amélioration d'adhérence cellulaire de telle sorte que ledit au moins un premier trou de chambre de ladite au moins une première cavité de chambre reste ouvert, la couche d'amélioration d'adhérence cellulaire étant composée d'un matériau hydrophile.

13. Appareil de culture cellulaire selon l'une quelconque des revendications 7 à 12, dans lequel chacun desdits au moins deux réservoirs de milieu de culture dans au moins un module de culture cellulaire desdits un ou plusieurs modules de culture cellulaire est mis en œuvre sous la forme d'un tube creux.

14. Appareil de culture cellulaire selon l'une quelconque des revendications 7 à 13, dans lequel au moins un module de culture cellulaire desdits un ou plusieurs modules de culture cellulaire comprend en outre au moins deux électrodes, dans lequel chacune des au moins deux électrodes est agencée indépendamment dans la première chambre, la structure de membrane de culture cellulaire (600) telle que définie dans la revendication 5, la seconde chambre, dans un ou plusieurs desdits au moins deux réservoirs de milieu de culture, ou la membrane, ou toute combinaison de ceux-ci.

15. Incubateur de culture cellulaire (1400) comprenant :
l'appareil de culture cellulaire (900) tel que défini dans l'une quelconque des revendications 7 à 14 ; et
une station de pipetage (1402) configurée pour alimenter en milieu de culture chacun desdits au moins deux réservoirs de milieu de culture dans chaque module de culture cellulaire desdits un ou plusieurs modules de culture cellulaire.

16. Procédé de production d'une structure de membrane de culture cellulaire, comprenant :
- le fait de prévoir une membrane (102, 402) faite en un matériau membranaire,
- le fait d'appliquer au moins une couche de revêtement (106, 108, 406, 408) sur la membrane (102, 402) d'une manière continue, ladite au moins une couche de revêtement (106, 108, 406, 408) étant composée d'un matériau de revêtement ; et
- le fait de former des pores traversants (104, 404) traversant ladite au moins une couche de revêtement (106, 108, 406, 408) et la membrane (102, 402) ;
dans lequel le matériau membranaire et le matériau de revêtement sont sélectionnés sur la base d'au moins un type de cellules à cultiver sur ladite au moins une couche de revêtement,
dans lequel la membrane composée d'un matériau membranaire comprend une première surface (110) qui est partiellement déformée par une ou plusieurs cavités (120) de structure de membrane faisant saillie depuis la première surface (110) ; ou
dans lequel le procédé comprend en outre, avant l'application d'au moins une couche de revêtement, avant la formation de pores traversants ou après la formation de pores traversants, le fait de déformer la membrane, obtenant ainsi une ou plusieurs cavités (120) de structure de membrane faisant saillie depuis une première surface de la membrane (110).

17. Procédé de culture cellulaire utilisant l'appareil de culture cellulaire telle que défini dans l'une quelconque des revendications 7 à 14, le procédé comprenant :
(a) le fait de fournir le milieu de culture à l'un desdits au moins deux réservoirs de milieu de culture dans chaque module de culture cellulaire desdits un ou plusieurs modules de culture cellulaire ; et
(b) le fait d'amener le milieu de culture à s'écouler depuis ledit un desdits au moins deux réservoirs de milieu de culture vers le reste desdits au moins deux réservoirs de milieu de culture dans chaque module de culture cellulaire desdits un ou plusieurs modules de culture cellulaire pendant une période de temps prédéfinie, la période de temps prédéfinie étant sélectionnée sur la base du milieu de culture.

18. Procédé de culture cellulaire utilisant l'appareil de culture cellulaire telle que défini dans l'une quelconque des revendications 7 à 14, le procédé comprenant :
(a) le fait de fournir le milieu de culture à un ou plusieurs desdits au moins deux réservoirs de milieu de culture dans chaque module de culture cellulaire desdits un ou plusieurs modules de culture cellulaire ;
(b) le fait d'amener le milieu de culture à s'écouler vers la seconde chambre depuis lesdits un ou plusieurs desdits au moins deux réservoirs de milieu de culture dans chaque module de culture cellulaire desdits un ou plusieurs modules de culture cellulaire ;
(c) le fait de placer l'appareil de culture cellulaire dans une position inversée ; et
(d) le fait d'incuber l'appareil de culture cellulaire dans la position inversée pendant une période de temps prédéfinie, la période de temps prédéfinie étant sélectionnée sur la base du milieu de culture.

19. Procédé selon l'une quelconque des revendications 17 à 18, comprenant en outre, pendant (b), le fait d'acheminer un autre milieu de culture via la première chambre ou la structure de membrane de culture cellulaire (600) telle que définie dans la revendication 5 vers la membrane dans au moins un module de culture cellulaire desdits un ou plusieurs modules de culture cellulaire.

20. Procédé selon l'une quelconque des revendications 17 à 19, dans lequel le milieu de culture comprend des cellules endothéliales vasculaires cérébrales et ledit autre milieu de culture comprend des astrocytes.
